# EUROPEAN PATENT APPLICATION

(11) **EP 4 616 878 A1**
(43) Date of publication of application: **17.09.2025**
(21) Application number: 24163955.8
(22) Date of filing: 15.03.2024
(51) Int. Cl.: A61M 3/02, A61K 9/00

(54) **CANNULA FOR RECTAL ADMINISTRATION OF AN ENEMA AND COMPOSITIONS FOR USE THEREWITH**

(71) Applicant: Cosmo Technologies Ltd., Dublin D02KV60 Dublin (IE)
(72) Inventor: LONGO, Luigi Maria, 20045 Milan (IT); PAGANI, Stefania, 20045 Milan (IT)
(74) Representative: FRKelly

(57) **Abstract**

The present disclosure relates to a cannula and an enema composition for rectal administration via the cannula, particularly a liquid enema composition that forms a gel at body temperature. The cannula and enema are suitable for use in the treatment of ulcerative colitis, particularly distal ulcerative colitis (including proctitis and proctosigmoiditis).

## Description

### BACKGROUND

The present disclosure relates to a cannula for rectal administration of an enema composition, an enema composition for rectal administration via the cannula, particularly a liquid enema composition that forms a gel at body temperature, and to the combination of said cannula and said enema composition. The cannula may be used for rectal administration of therapeutics. The cannula and enema composition are suitable for use in the treatment of ulcerative colitis, particularly distal ulcerative colitis, proctitis and proctosigmoiditis, or pouchitis.

Cannulas are typically used for dispensing medical fluid products and can be used to administer products to patients in various ways, such as, for example, vascularly, nasally, vaginally or rectally. Standard cannulas known in the art are composed of an elongate shaft, with an aperture present at the tip of the shaft (the proximal insertion end), through which the medical fluid is dispersed. Cannulas are generally tubular in shape, some being substantially cylindrical, while others may be frustoconical in shape i.e. a distal end of the cannula may be wider in cross-sectional diameter than the cross-sectional diameter of the proximal insertion end. The distal end may include a collar. Cannulas are typically made of plastic and may be designed for single or multiple use.

Rectal cannulas are typically used in conjunction with enemas, to administer an enema composition to the patient. The cannula shaft is placed inside the rectum, and the enema composition is administered via an aperture at the tip of the shaft. The enema composition is typically a liquid composition.

Standard rectal cannulas have a number of drawbacks, such as limited distribution of enema compositions. During administration the enema composition (a liquid or foam) is propelled from the proximal insertion end of the cannula and therefore does not achieve uniform distribution in the sigmoid colon, descending colon and particularly in the rectum. In particular, standard rectal cannulas, included in commercially available enemas, do not facilitate an optimal coating of the internal walls of the rectum, with most of the enema composition found in the sigmoid colon and, to a lesser extent, in the rectum. A clinical study published in literature investigated the spreading of mesalamine enema compositions (three volumes, 30 mL, 60 mL and 100 mL) in patients with mild to moderate ulcerative colitis; to precisely track the spread of the compositions following administration. The enema compositions were labelled with a radioactive tracer allowing for detection by scintigraphy. The study demonstrated that, in all the three groups, the percentage of the composition found in the rectum was less than 10%; notably, it was 1% and 0% in the 30 mL and 100 mL groups, respectively. While the 30 mL group did not reach the descending colon, due to limited volume, the enema composition reached the descending colon (25%) and sigmoid colon (66%) in the 100 mL group but failed to be delivered to the rectum (0%) (Van Bodegraven et al. Aliment Pharmacol Ther. 1996 (10): 327-332).

Unlike enemas, suppositories are active in the rectum only, and their effect does not extend to the sigmoid colon or the descending colon; therefore, their use is limited to patients who have inflammation in the rectum only (for example, patients with ulcerative proctitis) and not in the descending colon or the sigmoid colon.

Due to the poor distribution of the enema compositions in the rectum by standard rectal cannulas, some doctors combine the use of suppositories and of enemas in patients suffering from left-sided ulcerative colitis (also known as distal ulcerative colitis), which, per the Montreal classification of ulcerative colitis, involves the descending colon, the sigmoid colon and the rectum. By combining a suppository with an enema, a maximum effect is achieved in the rectum (due to the suppository) and in the descending colon and sigmoid colon (due to the enema composition); however, this combination therapy is disliked by patients, because it involves administering two rectal treatments rather than one. This leads to poor patient compliance with such a treatment regimen, especially in long-term use.

Further, standard enema compositions administered via standard rectal cannulas are liquid compositions, and therefore drip out of the patient's rectum following application. Thus, distribution is poor, and the liquid composition is wasted.

Ulcerative colitis (UC) represents, together with Crohn's disease, one of the main forms of IBD. UC represents a chronic disease characterized by inflammation and ulcers in the colon and rectum, which result in the main clinical symptoms, i.e. rectal bleeding, diarrhea, urgency, abdominal pain, tenesmus, and fever. Although the exact pathogenesis of UC is not completely understood, it is widely accepted within the scientific community that an altered interaction between the host's intestinal immune system and the commensal bacteria, normally colonizing the human colon, play a primary role in the pathogenesis of the disease. This altered interaction is exacerbated by an impaired barrier function of the epithelium lining the colonic mucosa, which leads the innate immune cells (e.g. dendritic cells and macrophages) to interact with the commensal microbiota and with their antigens. This results in an aberrant immune response, with consequent inflammation. At the onset, UC involves the rectum (ulcerative proctitis) in most cases; thereafter, the inflammation may extend proximally to involve the sigmoid colon (proctosigmoiditis) and descending colon up to the splenic flexure (distal UC, also known as left-sided UC). Inflammation extending beyond the splenic flexure is known as extensive UC (or pancolitis). A recent systematic literature review has estimated that, at diagnosis, about 40.1% of patients have distal UC, 30.5% have extensive colitis, and 29.4% have proctitis [Fumery et al., 2018].

Rectal treatments (e.g., enemas, foams or suppositories of mesalamine or corticosteroids) currently represent the standard of care for left-sided UC and of ulcerative proctitis, according to the guidelines on the management of UC published by the European Crohn's and Colitis Organisation (Raine et al. Journal of Crohn's and Colitis. 2022. 16(1): 2-17) and by the American College of Gastroenterology (Rubin et al. The American Journal of Gastroenterology. 2018. 114(3): 384-413). However, these rectal treatments are currently far from optimal, for the drawbacks highlighted above. Therefore, many doctors treat patients affected by left-sided UC by combining several rectal treatments, in order to achieve maximum effect in all the areas affected by inflammation.

A potentially curative surgical option for both ulcerative colitis (UC) and familial adenomatous polyposis (FAP) is a restorative proctocolectomy (RPC) preserving the anal sphincters, followed by an ileal pouch-anal anastomosis (IPAA), which recreates a faecal reservoir [Li and Shen, 2014]. Although this surgery has improved patient quality of life and significantly reduced the risk of dysplasia or neoplasia in ulcerative colitis patients, complications are common.

Pouchitis is an inflammatory disorder of the ileal pouch, the artificial rectum surgically created out of ileal gut tissue. It is the most common long-term complication of ileal pouch surgery and has a significant adverse impact on patient quality of life [Li and Shen, 2014]. About 50% patients with an IPAA for UC develop at least one episode of subsequent inflammation of the ileal pouch (pouchitis) that may evolve to a long-term complication [Li and Shen, 2014]. Contrarily, just 0-11% of FAP patients develop pouchitis. This data provides evidence that pouchitis is less related to the structure of the pouch but is a function of the patients' underlying immune dysregulation interacting with the pouch.

Pouchitis, similar to inflammatory bowel disease, is a complex disorder that is not caused by any one single factor. More likely, pouchitis occurs through a combination of both dysregulated host inflammatory mechanisms and interaction with luminal microbiota. Bacterial overgrowth and faecal stasis in the small intestine may contribute to the development of pouchitis. Thus, most patients with pouchitis are treated with antibiotics. The most frequently indicative symptoms of pouchitis are dysfunctions associated with increased frequency of evacuations, reduction in fecal consistency, rectorrhagia, pain, cramp-like abdominals, urgency, tenesmus, fecal incontinence, fever and extraintestinal manifestations. A clinical diagnosis should ideally be confirmed by endoscopy and mucosal biopsy of the pouch. Endoscopic examination shows inflammatory changes that may include mucosal edema, granularity, contact bleeding, loss of vascular pattern, hemorrhage, and ulceration. Histologic examination shows acute inflammation, including neutrophil infiltration and mucosal ulceration superimposed on a background of chronic inflammation including villous atrophy, crypt hyperplasia, and chronic inflammatory cell infiltration.

Al-Joufi, F., et al. (2021) discloses rectal mucoadhesive and in situ rectal gels containing, e.g., Rifampicin.

WO2019/122253 discloses a liquid composition that can be a structured viscous composition or a soft gel containing two or three defined polymers for in situ delivery or release of an active agent. The active agent may be antimicrobics or antibiotics selected from, among others, Rifamycin SV, Rifaximin, and Rifampicin. The liquid composition may be used in the diagnosis, prevention, alleviation, treatment and/or reduction of pathologies or disorders affecting the human body including those affecting the gastro-intestinal tract, such as disorders that are inflammatory and/or degenerative pathologies. Example 11 describes a liquid composition containing three defined polymers and rifamycin SV for treating fistulas.

Rifamycin SV is a semisynthetic antibacterial belonging to the ansamycin class; it exerts its antibacterial activity by irreversibly binding to the bacterial DNA-dependent RNA polymerase β-subunit, thereby inhibiting bacterial RNA synthesis. Rifamycin SV exhibits an antibacterial activity against most bacterial enteropathogens frequently associated with infectious diarrhoea and other enteric infections, including Enterobacteriaceae and non-Enterobacteriaceae [Clin Drug Investig 2019 Jul., 39(7):691-697]. Rifamycin SV corresponds to the sodium salt of Rifamycin or Rifamycin sodium. For colonic diseases like diverticulitis, inflammatory bowel syndrome (IBS) or inflammatory bowel disease (IBD), bacterial proliferation or microbial dysbiosis is associated with a strong inflammatory component. This inflammation has a profound influence on the liver via the gut-liver axis. Rifamycin is endowed with anti-inflammatory activities based on the impact on two key regulators of inflammation: pregnane X receptor (PXR) and nuclear factor-KB (NFκB). Rifamycin was found to activate PXR and two of its downstream targets, cytochrome P450 3A4 (CYP3A4) and P-glycoprotein (PgP), in liver and intestinal cell lines. Rifamycin also directly inhibited NFκB in a cell line which lacks PXR expression [Rosette, et al., 2019]. These dual activities likely explain the inhibition of pro-inflammatory cytokine secretion from human colonic cells lines and activated CD4+ T cells [Rosette, et al., 2013]. Rifamycin exerts its clinical benefit as topical action into the intestinal tract (J-Pouch, or S-pouch, or W-pouch in case of pouchitis) and it is characterized by a negligible absorption in the bloodstream. Rifamycin is a safe drug and has a favorable safety profile as compared to for example ciprofloxacin and metronidazole currently used in the treatment of gastrointestinal diseases with an inflammatory component, such as pouchitis, or proctitis, or ulcerative colitis.

WO2023/057499 discloses a pharmaceutical composition including a therapeutically effective amount of rifamycin SV, or a pharmaceutically acceptable salt thereof, and a polymer mixture, for use in treating pouchitis, proctitis and/or distal ulcerative colitis (including proctosigmoiditis), wherein the pharmaceutical composition is administered rectally, is a liquid at room temperature and forms a gel in situ in the patient.

Proctitis and/or distal ulcerative colitis are inflammatory diseases frequently associated to IBD in terms of symptoms and causes. Proctitis is the inflammation of the lining of the rectum. Depending on the cause, proctitis may happen suddenly and last a short time or may be long lasting with frequent relapsing episodes. As per IBD in general, bacterial overgrowth and/or dysregulation may be a worsening factor. Distal Ulcerative Colitis defines diseases distal to the splenic flexure which include proctitis (involvement of the rectum only), proctosigmoiditis (involvement of rectum and sigmoid colon) and left-sided colitis (involvement extending as far as descending colon or splenic flexure).

Currently, the clinical guidelines for the management of UC, including the American College Gastroenterology guideline (Rubin et al. The American Journal of Gastroenterology. 2018. 114(3): 384-413) and European Crohn's and Colitis Organisation guideline (Raine et al. Journal of Crohn's and Colitis. 2022. 16(1): 2-17), recommend using topical (rectal) treatments in patients with proctitis, proctosigmoiditis or distal UC. The first line treatment is represented by rectal products containing 5-aminosalycilic acid (also known as mesalamine or mesalazine) and the second line treatment is represented by rectal products containing corticosteroids (e.g. budesonide or hydrocortisone). These rectal treatments are available in the form of suppositories (used to treat proctitis) and enemas or rectal foams (used to treat proctosigmoiditis and distal UC). Conventional rectal cannulas dispense enema compositions via an apical aperture. This results in non-uniform coating of the enema composition in the rectum and sigmoid colon. During administration the liquid or foam enema composition is propelled from the proximal insertion end of the cannula, leading to significant application of the enema composition in the descending colon and the sigmoid colon, while the spreading of the compositions in the rectum is minimal. Therefore, as outlined above, many doctors treat patients affected by left-sided UC by combining several rectal treatments. Notwithstanding the foregoing combination treatment approach, compositions (both liquid and foam) leak from the application site and can be readily expelled by the patient following administration, with a consequent reduced contact time at the target site and loss of activity over time.

Mesalamine rectal suspension enema (4g/60 mL) is available from Encube Ethicals Private Limited. The suspension is delivered via a rectal applicator tip or cannula including an aperture at the tip of the proximal insertion end.

US8147445B2 discloses an enema and enema dispenser, the enema includes a liquid in a dispenser having a bottle, a nozzle attached to the bottle, and a valve. The valve may be a membrane having a slit and a thickness of at most 0.90 mm. The valve may be attached to the bottle or it may be attached to the nozzle. The enema can be administered more easily, with a lower amount of force. A method of bowel cleansing is also disclosed which includes inserting the enema into a rectum and applying a compression force to the enema bottle. The enema dispenser includes a nozzle (or cannula) including an apical aperture at the proximal insertion end thereof.
US20080215011 discloses an enema device for delivering an enema composition. The enema device includes a hollow elongated body having a first and second ends, a feed portion formed at the first end of the hollow elongated body, and an applicator portion disposed at the second end of the elongated body. The feed portion is in fluid communication with the interior of the hollow elongated body and is adapted to be placed into fluid communication with a source of enema fluid. The applicator portion defines a discharge opening in communication with the interior of the hollow elongated body. The device includes a "self-centering protrusion" including an apical aperture through which the enema is administered into the rectum.

CN212327085U discloses a device for administration of an enema. The device includes an enema bag, a catheter and an anal tube fixedly connected in sequence. The anal tube is the insertion end, and includes an apical orifice, with infusion holes circumferentially located close to the apical orifice. Multiple infusion holes are employed to reduce the likelihood of blockage associated with a single infusion hole.

CN209933787U discloses a glycerine enema with lubricating function. Figure 1 discloses a cannula including a tubular portion having an apical orifice with a plurality of holes provided in the sidewall of the tube. The presence of multiple holes avoids the likelihood of blockage associated with a cannula including a single dispensing orifice.

CN21157696U discloses a rectal lavage defecation device. The device includes a liquid outlet with three drainage pipe openings defined therein.

### SUMMARY

The present disclosure provides improved cannulas for rectal administration of an enema. The cannula is configured to ensure optimal coating of an enema composition within the rectum and colon. The cannula is particularly useful for administering therapeutics for treating diseases/conditions of the rectum and/or colon. For example, the cannula is particularly useful for administering enema compositions to the rectum and colon in order to treat ulcerative colitis, including distal ulcerative colitis (also known as left-sided colitis), ulcerative proctitis, and proctosigmoiditis.

The present disclosure provides, in some aspects, a cannula that achieves improved distribution of enema compositions, which may include increased coverage and reduced waste, and wherein the enema composition is administered to the rectum, sigmoid colon, and descending colon via the cannula. Cannula and enema compositions according to some aspects of the present disclosure are suitable for use in the treatment of ulcerative colitis, particularly distal ulcerative colitis, ulcerative proctitis and proctosigmoiditis.

The present disclosure provides, in some aspects, a cannula for rectal administration of an enema including: an elongate member having a lumen, said elongate member having a proximal insertion end and a distal end, and at least one lateral dispensing opening extending through a sidewall of the elongate member and configured to dispense fluid from the lumen at an angle of from about 30° to about 60° to a longitudinal axis of the elongate member.

By dispensing an enema composition at an angle of from about 30° to about 60° to a longitudinal axis (L) of the elongate member distribution of the enema within the rectum and colon can be improved. Some cannulas of the present disclosure have a dispensing angle from the lateral dispensing openings of from about 30° to about 60° to a longitudinal axis (L) of the elongate member application. This dispensing angle can significantly increase contact surface area in comparison to when an enema composition is administered rectally via a cannula including only an apical dispensing opening. Furthermore, the application contact surface area can also significantly increase when using a cannula according to some aspects of the present disclosure having a dispensing angle in the range of from about 30° to about 60° to a longitudinal axis (L) of the elongate member application, in comparison to the corresponding contact surface area when a cannula having a dispensing angle that is orthogonal (or substantially orthogonal) to a longitudinal axis of the cannula shaft is used.

The at least one lateral dispensing opening may be defined by a dispensing conduit in the sidewall of the elongate member (i.e. a lateral dispensing conduit), said dispensing conduit extending from an internal opening in an inner surface of the elongate member and an external opening in an outer surface of the elongate member, said dispensing conduit including a central axis extending at an angle of from about 30° to about 60° to a longitudinal axis of the elongate member.

The central axis may extend at an angle of from about 35° to about 55° to the longitudinal axis of the elongate member, in some embodiments from about 40° to about 50° to the longitudinal axis of the elongate member, such as from about 42° to about 48° to the longitudinal axis of the elongate member.

The cannula includes at least one lateral dispensing openings. For example, the cannula may include two lateral dispensing openings. The two lateral dispensing openings may or may not be offset with respect to each other along the longitudinal axis of the elongate member. In some embodiments, the cannula may include two or more lateral dispensing openings offset with respect to each other along the longitudinal axis of the elongate member. The number of the lateral dispensing openings may vary depending on the volume of composition, which is intended to be delivered in the rectum. In some embodiments, the cannula comprises one lateral dispensing opening. In another embodiment, the cannula comprises two lateral dispensing opening. In another embodiment, the cannula comprises three lateral dispensing openings. In another embodiment, the cannula comprises four lateral dispensing openings. In another embodiment, the cannula comprises five lateral dispensing openings. In another embodiment, the cannula comprises six lateral dispensing openings. In another embodiment, the cannula comprises seven lateral dispensing openings. In another embodiment, the cannula comprises eight lateral dispensing openings. In some embodiments, the cannula comprises exactly two lateral dispensing openings.

Suitably, the cannula may include an apical dispensing opening in a tip of the proximal insertion end.

Optionally, the external opening of the at least one lateral dispensing opening is circular or non-circular in circumference, in some embodiments, the external opening of at least one lateral dispensing opening is non-circular in circumference.

Suitably, the lateral dispensing conduit may be polygonal in cross-section when viewed parallel to the longitudinal axis of the elongate member. For example, the lateral dispensing conduit may be quadrilateral in cross-section, optionally, rectangular in cross-section when viewed parallel to the longitudinal axis of the elongate member.

Suitably, at least one lateral dispensing opening has an internal opening in the inner surface of the elongate member having a circumference greater in length than a circumference of the external opening in the outer surface of the elongate member.

Optionally, at least one lateral dispensing opening forms a nozzle through the sidewall extending from the lumen, optionally, wherein the nozzle is a converging nozzle.

The lateral dispensing conduit may have a cross-sectional area when viewed parallel to the longitudinal axis of the elongate member in the range of from about 0.5 mm² to about 5 mm², such as from about 1 mm² to about 4.5 mm², optionally from about 1.5 mm² to about 3 mm², for example about 2.4 mm².

The elongate member may be from about 3 cm to about 10 cm in length, suitably, the elongate member is from about 3.5 cm to about 8 cm in length, optionally, from about 4 cm to about 6 cm in length.

Optionally, at least one lateral dispensing opening is 0.5 cm to 2.5 cm from the tip of the proximal insertion end, optionally, wherein at least one lateral dispensing opening is 1 cm to 2 cm from the tip of the proximal insertion end.

The elongate member may be substantially frustoconical in shape.

Optionally, the proximal insertion end has an external diameter of from 4.5 mm to 7.5 mm in length, optionally of from 5 mm to 7 mm in length, such as from 5.5 mm to 6.5 mm in length.

Optionally, the distal end of the elongate member has an external diameter of from 7 mm to 14 mm in length, optionally from 8 mm to 12 mm in length, such as from 9 mm to 11 mm in length.

Suitably, the cannula further comprises a reservoir. Optionally, the distal end of the elongate member includes attachment means for attaching the cannula to a reservoir.

Suitably, the cannula further includes a reservoir in fluid connection with the lumen of the elongate member.

The reservoir may include an enema composition. Suitably, the enema composition is a liquid at room temperature and forms a gel at body temperature i.e. the enema composition includes an in-situ gelling enema composition. In some embodiments, the enema composition includes a therapeutic.

In further embodiments, the enema composition is suitable for treating diseases or conditions of the rectum and/or colon. Suitably, the disease of the rectum and/or colon are selected from inflammatory bowel disease (IBD), inflammatory bowel syndrome (IBS), diverticulitis, small intestinal bacterial overgrowth (SIBO), ulcerative colitis, pouchitis, proctitis, proctosigmoiditis, left-sided colitis (also known as distal ulcerative colitis), pancolitis, fecal incontinence, ulcerative colitis, particularly distal ulcerative colitis, proctitis and proctosigmoiditis, or pouchitis. In an aspect, the enema composition is suitable for treating ulcerative colitis. In another aspect, the enema composition is suitable for treating left-sided colitis (also known as distal ulcerative colitis). In another aspect, the enema composition is suitable for treating proctosigmoiditis. In another aspect, the enema composition is suitable for treating proctitis.

Suitably, the enema composition can include a therapeutic agent and a polymeric mixture. Suitably, the enema composition can be able to gel in-situ upon contact with the target site of action ensuring the prolongation of the contact time with the mucosa therein ensuring a sustained release of the active agent topically over time. This means that the enema composition upon application on the intestinal mucosa can become a long-lasting gel acting at the targeted site.

Optionally, the polymeric mixture includes at least one thermo-responsive polymer (first polymer) and at least one ion-sensitive polymer (second polymer). Optionally, the polymeric mixture further comprises at least one bio-adhesive polymer (third polymer).

Optionally, the first polymer is selected from the group including but not limited to: polyoxyethylene-polyoxypropylene block copolymers, such as poloxamer 124, poloxamer 188, poloxamer 237, poloxamer 338, poloxamer 407 and the like, polyethylene glycol)/poly(lactide-coglicolide) block copolymers (PEG-PLGA), polyethylene glycol)-poly(lactic acid)-poly(ethylene glycol) (PEG-PLA-PEG), poly(N-isopropylacrylamide), and cellulose derivatives, like methylcellulose (MC) and hydroxypropylmethylcellulose (HPMC) and the like and mixtures thereof. In some embodiments, the first polymer is a polyoxyethylene-polyoxypropylene block copolymer selected from the group consisting of: poloxamer 124, poloxamer 188, poloxamer 237, poloxamer 338, and poloxamer 407; or a cellulose derivative selected from the group consisting of methylcellulose (MC), hydroxypropylmethylcellulose (HPMC) and mixtures thereof. In some embodiments, the first polymer is polyoxamer 188, poloxamer 407, or mixtures thereof.

Optionally, the second polymer is a polysaccharide selected from the group including but not limited to: carrageenan, gellan gum, pectin, alginate, alginate salts and the like, alginic acid, and mixtures thereof. In some embodiments, the second polymer is sodium alginate.

Optionally, the third polymer is selected from the group including, but not limited to: chitosan, hyaluronic acid and salts thereof, cellulose derivatives, such as methyl cellulose, hydroxy propyl methylcellulose, hydroxy propyl cellulose, carboxymethyl cellulose sodium and the like, polyvinyl alcohol, polyvinyl pyrrolidone, polyacrylic acid, polyethylene oxides, cyclodextrins, tragacanth, sodium alginate, xanthan gum, gelatin, pectin, and the like and mixtures thereof. In some embodiments, the third polymer is a cellulose derivative selected from the group consisting of: methyl cellulose, hydroxy propyl methylcellulose, hydroxy propyl cellulose, carboxymethyl cellulose sodium and mixtures thereof. In some embodiments, the third polymer is a sodium salt of carboxymethyl cellulose.

Optionally, the at least one thermo-responsive polymer is present in an amount of from about 1% to about 25 % by weight with respect to the weight of the enema composition. In some embodiments, the at least one thermo-responsive polymer is present in an amount from about 5% to about 22% by weight with respect to the weight of the enema composition. In some embodiments, the at least one thermo-responsive polymer is present in an amount from about 10% to about 20% by weight with respect to the weight of the enema composition. In some embodiments, the at least one thermo-responsive polymer is present in an amount from about 12% to about 18% by weight with respect to the weight of the enema composition.

Optionally, the at least one ion-sensitive polymer is present in an amount of from about 0.01% to about 3% by weight with respect to the weight of the enema composition. In some embodiments, the at least one ion-sensitive polymer is present in an amount of from about 0.05% to about 2.5 % by weight with respect to the weight of the enema composition. In some embodiments, the at least one ion-sensitive polymer is present in an amount of from about 0.1% to about 2.0 % by weight with respect to the weight of the enema composition.

Optionally, the at least one bio-adhesive polymer is present in an amount of from about 0.01% to about 2.0% by weight with respect to the weight of the enema composition. In some embodiments, the at least one bio-adhesive polymer is present in an amount of from about 0.02% to about 1.5% by weight with respect to the weight of the enema composition. In some embodiments, the at least one bio-adhesive polymer is present in an amount of from about 0.03% to about 1.0% by weight with respect to the weight of the enema composition. In some embodiments, the at least one bio-adhesive polymer is present in an amount of from about 0.04% to about 0.5% by weight with respect to the weight of the enema composition. In some embodiments, the at least one bio-adhesive polymer is present in an amount of from about 0.05% to about 0.1% by weight with respect to the weight of the enema composition.

Optionally, the enema composition further includes at least one other excipient such as for example antioxidants, chelating agents, preservatives, antimicrobial agents, surfactants, co-surfactants, lipophilic compounds, purified water, organic salts, inorganic salts, buffers agents or mixtures thereof. In some embodiments, the enema composition further includes at least one antioxidant and a least one preservative.

Optionally, the enema composition further includes at least one therapeutic agent (or active substance) and at least one physiologically acceptable excipient.

Suitably, the therapeutic agent can be selected from the therapeutic classes comprising, but not limited to: aminosalycilates, corticosteroids, immunomodulators, tumor necrosis factor alpha (TNFα) inhibitors, integrin antagonists, interleukin antagonists, janus kinase (JAK) inhibitors, sphingosine 1 phosphate (S1p) modulators, oligonucleotides, antibiotics and probiotics.

In the therapeutic class of non-steroidal aminosalycilates, suitable therapeutic agents for the present disclosure provided can be selected in the group comprising: mesalamine (also known as mesalazine or 5-aminosalycilic acid), sulfasalazine, olsalazine, balsalazine, and the like.

In the therapeutic class of corticosteroids, suitable therapeutic agents for the present disclosure provided can be selected in the group comprising: betamethasone and salts thereof, hydrocortisone and salts thereof, budesonide, prednisone, prednisolone, methylprednisolone, beclomethasone and salts thereof, and the like.

In the therapeutic class of immunomodulators, suitable therapeutic agents for the present disclosure provided can be selected in the group comprising: azathioprine, cyclosporine A, 6-mercaptopurine, tacrolimus, and the like.

In the therapeutic class of tumor necrosis factor alpha (TNFα) inhibitors, suitable therapeutic agents for the present disclosure provided can be selected in the group comprising: infliximab, adalimumab, certolizumab pegol, golimumab and the like.

In the therapeutic class of integrin antagonists, suitable therapeutic agents for the present disclosure provided can be selected in the group comprising: natalizumab, vedolizumab and the like.

In the therapeutic class of interleukin antagonists, suitable therapeutic agents for the present disclosure provided can be selected in the group comprising: ustekinumab, risankizumab, mirikizumab and the like.

In the therapeutic class of janus kinase (JAK) inhibitors, suitable therapeutic agents for the present disclosure provided can be selected in the group comprising: tofacitinib, upadacitinib, filgotinib and the like.

In the therapeutic class of sphingosine 1 phosphate (S1p) modulators, suitable therapeutic agents for the present disclosure provided can be selected in the group comprising: ozanimod, estrasimod and the like.

In the therapeutic class of oligonucleotides, suitable therapeutic agents for the present disclosure provided can be selected in the group comprising: alicaforsen, cobitolimod and the like.

In the therapeutic class of antibiotics, suitable therapeutic agents for the present disclosure provided can be selected in the group comprising: penicillins (e.g., amoxicillin, ampicillin, dicloxacillin, oxacillin, piperacillin and the like), tetracyclines (democycline, doxycycline, eravacycline, minocycline, omadacycline, sarecycline, tetracycline and the like) cephalosporines (cefaclor, cefadroxil, cefdinir, cephalexin, cefprozil, cefdinir, cefepime, cefiderocol, cefotaxime, cefotetan, ceftaroline, ceftazidime, ceftriaxone, cefuroxime and the like), fluoroquinolones (ciprofloxacin, delafloxacin, levofloxacin, moxifloxacin, gemifloxacin and the like), lincomycines (clindamycin, lincomycin and the like), macrolides (azithromycin, clarithromycin, erythromycin, fidaxomicin and the like), sulfonamides (sulfamethoxazole, succinylsulfathiazole, sulfisoxazole and the like), glycopeptide antibiotics (dalbavancin, oritavancin, telavancin, vancomycin and the like), aminoglycosides (gentamicin, tobramycinm, amikacin and the like), carbapenems (imipenem, meropenem, ertapenem and the like), nitroimidazoles (metronidazole, tinidazole, nimorazole and the like), ansamycines (rifamycin SV or salts thereof, rifaximin, rifabutin, rifapentine, rifampicin and the like). Optionally, the therapeutic is rifamycin SV or salts thereof.

In some aspects, the enema composition contains more than one therapeutic agents, selected from the groups listed hereinabove.

In some aspects, the therapeutic agent is an aminosalicylate and is selected from mesalamine or olsalazine. Optionally, the therapeutic agent is mesalamine.

In other aspects, the therapeutic agent is a corticosteroid and is selected from busedonide, bethametasone or salts thereof, or prednisone. Optionally, the therapeutic agent is budesonide.

In other aspects, the therapeutic agent is an immunomodulators, and is selected from 6-mercaptopurine and cyclosporine A. Optionally, the therapeutic agent is cyclosporine A.

In other aspects, the therapeutic agent is a TNFα inhibitor and is selected from infliximab or adalimumab. Optionally, the therapeutic agent is infliximab.

In other aspects, the therapeutic agent is an integrin antagonist and is selected from natalizumab and vedolizumab. Optionally, the therapeutic agent is vedolizumab.

In other aspects, the therapeutic agent is an interleukin antagonist and is selected from ustekinumab and mirikizumab. Optionally, the therapeutic agent is mirikizumab.

In other aspects, the therapeutic agent is a janus kinase (JAK) inhibitors, selected from tofacitinib and upadacitinib. Optionally, the therapeutic agent is tofacitinib.

In other aspects, the therapeutic agent is an S1p modulator and is selected from ozanimod and estrasimod. Optionally, the therapeutic agent is ozanimod.

In other aspects, the therapeutic agent is an olignucleotide and is alicaforsen.

In other aspects, the therapeutic agent is an antibiotic and is selected from ciprofloxacin, metronidazole, amoxicillin, vancomycin, rifaximin or rifamycin SV or salts thereof.

In some embodiments, the therapeutic agent is rifamycin SV or salts thereof.

The present disclosure also relates to a method of treating ulcerative colitis, particularly distal ulcerative colitis, proctitis and proctosigmoiditis, or pouchitis by administering an in-situ gelling enema composition using a cannula as described herein.

The present disclosure provides a method of treating ulcerative colitis, particularly distal ulcerative colitis, proctitis and proctosigmoiditis, or pouchitis including employing a cannula as defined herein to rectally administer to a patient in need thereof, an enema composition including a therapeutic agent and a polymeric mixture, wherein the therapeutic agent includes rifamycin SV, or a pharmaceutically acceptable salt thereof, and wherein the enema composition is a liquid at room temperature and forms a gel in situ at body temperature of the patient.

Optionally, the enema composition has a pH in the range of from 6.0 to 8.0, or in the range of from 6.5 to 7.5, or in the range of from pH 6.8 to 7.2.

Optionally, the enema composition is administered to a patient in need thereof daily or twice a day.

Optionally, the enema composition is administered for at least two weeks.

Optionally, the enema composition is self-administered by the patient in need thereof using the rectal cannula described herein.

According to a further aspect, there is provided a kit of parts comprising:
(i) a first package enclosing a cannula as defined herein, and
(ii) a second package encasing a reservoir (e.g. a container) containing an enema composition.

According to a further aspect, there is provided a kit of parts comprising:
(i) a first package enclosing a cannula as defined herein, and
(ii) a second gas impermeable package encasing a reservoir containing an enema composition, wherein optionally, the second package is an aluminum pouch.

According to a further aspect, there is provided a kit of parts comprising:
(iii) a first package enclosing a cannula as defined herein, and
(iv) a second gas impermeable package encasing: (a) a reservoir including an enema composition, and (b) an oxygen scavenger, wherein optionally, the second package is an aluminum pouch.

For example, the kit may include:
(i) a package enclosing a cannula as defined herein; and
(ii) an aluminum pouch encasing (a) a reservoir including an enema composition as defined herein, and (b) an oxygen scavenger.

Suitably, the reservoir is a bottle, such as a bellows bottle, optionally, the bottle is sealed with a cap. Optionally, the cap is a screw cap.

Optionally, the kit is provided in an outer packaging, such as a cardboard or plastic box.

According to a further aspect of the present disclosure, there is a provided a method of treating, ameliorating, reducing the severity of, slowing the progression of, inducing the remission of, or maintaining the remission of ulcerative colitis, such as distal ulcerative colitis, proctitis and/or proctosigmoiditis, including employing a cannula as defined herein to rectally administer an enema composition to a patient in need thereof, wherein the enema composition is a liquid composition at room temperature and forms a gel at body temperature.

According to a further aspect of the present disclosure, there is a provided a method of treating, ameliorating, reducing the severity of, slowing the progression of, inducing the remission of, or maintaining the remission of pouchitis, including employing a cannula as defined herein to rectally administer an enema composition to a patient in need thereof, wherein the enema composition is a liquid composition at room temperature and forms a gel at body temperature.

In some embodiments, the liquid enema composition includes a therapeutically effective amount of rifamycin SV, or a pharmaceutically acceptable salt thereof.

Optionally, rectally administering the enema composition to the patient in need thereof using the rectal cannula described herein alleviates and/or reduces one or more clinical symptoms selected from the group consisting of: increased frequency of evacuations, reduction in fecal consistency, rectorrhagia, pain, cramp-like abdominals, urgency, tenesmus, fecal incontinence, fever and extraintestinal manifestations.

Optionally, rectally administering the enema composition to the patient in need thereof using the rectal cannula described herein alleviates and/or reduces the occurrence of one or more endoscopy symptoms selected from the group consisting of: mucosal edema, granularity, contact bleeding, loss of vascular pattern, hemorrhage, and ulceration.

In a still further aspect, the present disclosure provides a method of administering an enema composition, the method comprising:
a. disposing the cannula as described herein at a dispensing position within a patient's rectum, wherein
   cannula is assembled with a reservoir of the enema composition such that the enema composition may travel from the reservoir into the lumen; and
b. dispensing the enema composition from the apical and the at least one lateral dispensing opening while the cannula remains at the dispensing position.

Suitably, the enema composition is stored as a fluid in the reservoir at a temperature below body temperature prior to the disposing step and the enema composition is configured to transition from the fluid to a gel at body temperature.

Suitably, the at least one lateral dispensing opening is located at about 3 cm to 6 cm from the patient's anal verge when the cannula is at the dispensing position (within the rectum).

### BRIEF DESCRIPTION OF THE FIGURES

The drawings directly relate to the Examples set out herein and are explained in detail later in this document.
FIG. 1A is a partially cut-away side elevation view of a cannula according to a first aspect of the present disclosure.
FIG. 1B is a partially cut-away side elevation view of a further cannula according to the first aspect of the present disclosure.
FIG. 1C is a partially cut-away side elevation view of a further cannula according to the first aspect of the present disclosure.
FIG 1D is a partially cut-away side elevation view of a further cannula according to the first aspect of the present disclosure.
FIG. 1E is a partial cut-away side elevation view of a further cannula according to the first aspect of the present disclosure.
FIG. 2 is a cross-sectional view of a portion of a cannula according to a second aspect of the present disclosure.
FIG. 3A is a cross-sectional view of a further cannula according to the second aspect of the present disclosure.
FIG. 3B is a cross-sectional view of a further cannula according to the second aspect of the present disclosure.
FIG. 4 is a side elevation view of a cannula according to a third aspect of the present disclosure.
FIGs. 5A and 5B schematically represent stages of assembling a cannula together with a reservoir according to a fourth aspect of the present disclosure.
FIG. 6A and 6B illustrate positions for administration of an enema.
FIG. 7 illustrates a cannula according to some aspects of the present disclosure disposed at a dispensing position with respect to a colon.
FIG. 8A is a plot of viscosity against shear rate of an enema composition according to some aspects of the present disclosure in a liquid state and in a gel state.
FIG. 8B is a plot of G' and G" against temperature of enema compositions according to some aspects of the present disclosure.

### DEFINITIONS

References in the specification to "one embodiment", "an embodiment", "one aspect", "an aspect" and similar indicate that the described embodiment or aspect may include a particular aspect, feature, structure or characteristic. Moreover, such phrases may, but do not necessarily, refer to the same embodiment or aspect referred to in other portions of the specification. Further, when a particular aspect, feature, structure or characteristic is described in connection with an embodiment or aspect, it is within knowledge of a person skilled in the art to affect or connect said aspect, feature, structure or characteristic with other embodiment or aspect, whether or not explicitly described. The singular forms "a", "an" and "the" include plural references unless the context clearly dictates otherwise. Thus, for example, a reference to "a compound" includes a plurality of such compounds. It is further noted that the claims may be drafted to exclude any optional element. As such, this statement is intended to serve as antecedent basis for the use of exclusive terminology, such as "solely", "only", and the like, in connection with the recitation of claims elements or use of a "negative" limitation.

The term "and/or" means anyone of the items, any combination of the items, or all the items with which this term is associated.

The terms "comprising", "having", "including" and "containing" are to be construed as open-ended terms (i.e. meaning "including, but not limited to") and are to be considered as providing support also for terms as "consist essentially of", "consisting essentially of", "consist of' or "consisting of".

The terms "consist essentially of", "consisting essentially of" are to be construed as a semi-closed terms, meaning that no other ingredients which materially affects the basic and novel characteristics (and optionally physiologically acceptable excipients and/or adjuvants) of the disclosure are included.

The terms "consists of", "consisting of' are to be construed as a closed term.

The term "polymer mixture" is intended to mean the enema composition without the active agent. Thus, in its simplest usage, the "polymer mixture" includes water or other suitable carrier for the formulation of an enema, the at least one thermo-responsive polymer, the at least one ion-sensitive polymer and the at least one bio-adhesive polymer, as well as any excipients described herein.

Unless indicated otherwise herein, the term "about" is intended to include values, e.g. weight percentages, proximate to the recited range that are equivalent in terms of the functionality of the individual ingredient, the composition, or the embodiment.

A person skilled in the art will recognize that, for any and all purposes, particularly in terms of providing a written description, all ranges recited herein also encompass any and all possible sub-ranges and combinations of sub-ranges thereof, as well as the individual values making up the range, particularly integer values. A recited range includes each specific value, integer, decimal, or identity within the range.

A person skilled in the art will recognize that where members are grouped together in a common manner, such as in a Markush group, the disclosure encompasses not only the entire group listed as a whole, but each member of the group individually and all possible subgroups of the main group. Additionally, for all purposes, the disclosure encompasses not only the main group, but also the main group absent one or more of the group members. The disclosure therefore envisages the explicit exclusion of anyone or more of members of a recited group. Accordingly, provisos may apply to any of the disclosed categories or embodiments whereby anyone or more of the recited elements, species, or embodiments, may be excluded from such categories or embodiments, for example, as used in an explicit negative limitation.

The term "alleviating" refers to relieving the symptoms and/or manifestations of inflammatory and/or degenerative diseases of the gastrointestinal tract.

The term "reducing" refers to decreasing the extent of the damage caused by inflammatory and/or degenerative diseases of the gastrointestinal tract or to decreasing clinical signs or symptoms associated with such damage.

The "viscosity" defines the resistance of a liquid or semisolid against flow. The flow of liquids or semisolids is described by viscosity, or, more precisely, by shear viscosity η. The shear viscosity of a fluid expresses its resistance to shearing flows, where adjacent layers move parallel to each other with different speeds. Common units of measurement of viscosity are the pascal-second (Pas), the poise (P) and cP (centipoises).

"Modulus G'" refers to elastic or storage modulus obtained in dynamic regimen. An elastic modulus (also known as modulus of elasticity) is a number that measures an object or substance's resistance to being deformed elastically (i.e., non-permanently) when a stress is applied to it. The elastic modulus of an object is defined as the slope of its stress-strain curve in the elastic deformation region.

"Body temperature" refers to the level of heat produced and sustained by the body processes. Heat is generated within the body through metabolism of nutrients and lost from the body surface through radiation, convection, and evaporation of perspiration. Heat production and loss are regulated and controlled in the hypothalamus and brainstem. Normal adult body temperature, as measured orally, is 37 °C even though little variations are normally recorded throughout the day.

"Room temperature" (RT) is generally defined as the ambient air temperature in whatever environment being used for a given procedure. More specifically, it is defined as 20-25 °C., as some ambient temperatures, by nature, do not fall within this range. Generally, protocols calling for steps to be performed at RT require that temperatures do not fall below 18 °C, and do not exceed 27 °C.

"MIC" refers to Minimum Inhibitory Concentration and it is defined as defined as the lowest concentration expressed in mg/L (equivalent to µg/mL) of an antimicrobial ingredient or agent that is bacteriostatic (prevents the visible growth of bacteria).

"Rifamycin SV" generally refers to Rifamycin itself or salts thereof, such as Rifamycin SV sodium salt defined to the CAS N° 14897-39-3. However, where quantities or proportions are described herein, the term "Rifamycin SV" refers strictly to the active moiety of Rifamycin. Thus, a reference herein to a solution of 1.0% concentration Rifamycin SV indicates a 1.0% concentration of the active moiety, regardless of the possibly greater proportion by weight of Rifamycin salt that may have been included in the solution. Other than for the description of quantities and proportions, the term "Rifamycin SV" shall not exclude Rifamycin salts.

The term "therapeutically effective amount" refers to the amount of a compound which, upon administration to a subject in need thereof, is sufficient to treat the disease. In this case, the therapeutically effective amount of Rifamycin SV is the amount of Rifamycin SV required to treat, ameliorate, reduce the severity of, slow the progression of, induce the remission of, or maintain the remission of pouchitis and/or proctitis and/or distal ulcerative colitis (including proctosigmoiditis) when administered to a patient in need thereof. The term "long-lasting" means that the formulation remains in the site of action for the time necessary to exert the therapeutic effect.

As used herein with respect to cannulas and associated enema tools, the terms "proximal" and "distal" refer to opposite directions defined with respect to the tools such that the apical tip of the cannula is the proximal-most extreme of the tools, while features of the tools are relatively more distal with increasing distance from the apical tip of the cannula. Further, objects beyond the apical tip in a direction away from distal features of the tool shall be considered beyond the cannula in the proximal direction defined with respect to the tools. As used herein with respect to the patient's anatomy, the term "proximal" refers to features nearer to the center of the trunk of the patient's body and the term "distal" refers to features farther from the center of the trunk of the patient's body. Thus, when a cannula according to the present disclosure is disposed at an intended position for dispensing enema composition, the term "proximal" may refer to the same direction whether used with respect to features of the cannula or features of the patient's anatomy.

Terms of approximation, such as "approximately" and "about," when applied to a quantity refer to a range extending from 10% less than the stated quantity to 10% more than the stated quantity. In any instance where a term of approximation is used with respect to a stated quantity, the inventors have contemplated embodiments having precisely the stated quantity. Thus, for example, a reference herein to "a length of about 1.0 mm" would allow for the length to be in a range extending from 0.9 mm to 1.1 mm while indicating that the inventors have contemplated that the length may be exactly 1.0 mm.

### DETAILED DESCRIPTION

The present disclosure relates to a cannula for rectal administration of an enema, and an enema composition for rectal administration via the cannula, particularly a liquid enema composition that forms a gel at body temperature. The cannula and enema composition are particularly useful for administering/delivering enema compositions for treating diseases conditions of the rectum and lower colon.

The cannula and enema are suitable for use in the treatment of ulcerative colitis, particularly distal ulcerative colitis, proctitis and proctosigmoiditis.

As intended herein, the term distal ulcerative colitis (also known as left-sided ulcerative colitis) also includes ulcerative proctosigmoiditis (or proctosigmoiditis, by simplicity), and proctitis. The cannula and enema composition disclosed herein may also be used for treating pouchitis.

The present disclosure provides a cannula for rectal administration of an enema including: an elongate member having a lumen, said elongate member having a proximal insertion end and a distal end, and at least one lateral dispensing opening extending through a sidewall of the elongate member and configured to dispense fluid from the lumen at an angle of from about 30° to about 60° to a longitudinal axis of the elongate member. Advantageously, a dispensing angle in this range can contribute to optimal coating of the sidewalls of the rectum. Such delivery may be achieved by at least one lateral dispensing opening defined by dispensing conduit in the sidewall of the elongate member, said dispensing conduit extending from an internal opening in an inner surface of the elongate member and an external opening in an outer surface of the elongate member, said dispensing conduit including a central axis extending at an angle of from about 30° to about 60° to a longitudinal axis of the elongate member. In some embodiments, the cannula includes a plurality of lateral dispensing openings defined in the sidewalls of the elongate member. For example, the cannula may include two or more lateral dispensing openings offset with respect to each other in the longitudinal axis of the elongate member. In some embodiments, the cannula includes an apical dispensing opening in a tip of the proximal insertion end. In further embodiments, the cannula includes two lateral dispensing openings and one apical dispensing opening.

The cannula includes at least one lateral dispensing openings. In some embodiments, the cannula includes two or more lateral dispensing openings, which lateral dispensing openings may or may not be offset with respect to each other along the longitudinal axis of the elongate member. In some embodiments, the cannula may include two or more lateral dispensing openings offset with respect to each other in the longitudinal axis of the elongate member. The number of the lateral dispensing openings may be varied depending on the volume of composition, which is intended to be delivered in the rectum. In some embodiments, the cannula comprises one lateral dispensing opening. In another embodiment, the cannula comprises two lateral dispensing opening. In another embodiment, the cannula comprises three lateral dispensing openings. In another embodiment, the cannula comprises four lateral dispensing openings. In another embodiment, the cannula comprises five lateral dispensing openings. In another embodiment, the cannula comprises six lateral dispensing openings. In another embodiment, the cannula comprises seven lateral dispensing openings. In another embodiment, the cannula comprises eight lateral dispensing openings. In some embodiments, the cannula comprises exactly two lateral dispensing openings.

Optionally, the external opening of the at least one lateral dispensing opening is circular or non-circular in circumference, suitably, the external opening of at least one lateral dispensing opening is non-circular in circumference. Suitably, the external openings of the lateral dispensing openings can be smoothened, and/or softened to reduce discomfort to the patient, and reduce the likelihood of abrasion on insertion and removal of the cannula.

The dispensing conduit may be polygonal in cross-section when viewed parallel to the longitudinal axis of the elongate member. For example, the lateral dispensing conduit may be quadrilateral in cross-section, optionally, rectangular in cross-section when viewed parallel to the longitudinal axis of the elongate member.

Suitably, at least one lateral dispensing opening can have an internal opening in the inner surface of the elongate member having a circumference greater in length than a circumference of the external opening in the outer surface of the elongate member. In such a configuration the at least one dispensing conduit forms a nozzle, specifically a converging nozzle, through the sidewall of the elongate member extending from the lumen. This can advantageously focus the stream of fluid dispensed from the cannula, ensuring a deeper application to the sidewalls and upper sections of the rectum from the lateral dispensing openings.

Suitably, the lateral dispensing conduit has a cross-sectional area when viewed parallel to the longitudinal axis of the elongate member in the range of from about 0.5 mm² to about 5 mm², such as from about 1 mm² to about 4.5 mm², optionally from about 1.5 mm² to about 3 mm², for example about 2.4 mm².

Suitably, the apical dispensing opening has a cross-sectional area in the range of from 4 mm² to 12 mm², optionally, from 5 mm² to 10 mm², such as from 5 mm² to 9 mm², for example about 7 mm².

Optionally, the lateral dispensing conduit has a cross-sectional area when viewed parallel to the longitudinal axis of the elongate member in the range of from about 1 mm² to about 4.5 mm², optionally from about 1.5 mm² to about 3 mm², for example about 2.4 mm², and the apical dispensing opening has a cross-sectional area in the range of from 4 mm² to 12 mm², and in some embodiments, from 5 mm² to 10 mm², such as from 5 mm² to 9 mm². Suitably, the lateral dispensing conduit has a cross-sectional area when viewed parallel to the longitudinal axis of the elongate member of about 2.4 mm² and the apical dispensing opening has a cross-sectional area of about 7 mm².

Suitably, the elongate member can be from about 3 cm to about 10 cm in length, optionally from about 3.5 cm to about 8 cm in length, in some embodiments from about 4 cm to about 6 cm in length.

Suitably, at least one lateral dispensing opening can be 0.5 cm to 2.5 cm from the tip of the proximal insertion end, optionally, wherein at least one lateral dispensing opening is 1 cm to 2 cm from the tip of the proximal insertion end.

Optionally, two lateral dispensing openings are disposed 1 cm to 2 cm from the tip of the proximal insertion end.

Suitably, the elongate member can be substantially frustoconical in shape. Suitably, the elongate member can be tapered at an angle of from 0.5 to 15 degrees, suitably, from 1 to 10 degrees, such as from 3 to 8 degrees. Optionally, the elongate member is tapered from the proximal insertion end to the distal end at an angle of from 0.5 to 15 degrees, suitably, from 1 to 10 degrees, such as from 3 to 8 degrees.

Suitably, the proximal insertion end can have an external diameter of from 4.5 mm to 7.5 mm in length, optionally of from 5 mm to 7 mm in length, such as from 5.5 mm to 6.5 mm in length.

Suitably, the distal end of the elongate member can have an external diameter of from 7 mm to 14 mm in length, optionally from 8 mm to 12 mm in length, such as from 9 mm to 11 mm in length.

The distal end of the elongate member can suitably include attachment means for attaching the cannula to a reservoir. In some embodiments the attachment means can be, for example, threading configured to engage a corresponding threaded feature of the reservoir. For example, part of the distal end of the elongate member can be internally threaded, and the reservoir can have a complementary externally threaded portion configured for threaded engagement with the internal threading of the distal end of the elongate member. The reservoir is suitably in fluid connection with the lumen of the elongate member.

The cannula described herein can suitably be formed of a polymeric material optionally, polyethylene, polypropylene, polyethylene terephthalate, polyurethane or polyvinyl chloride. In some embodiments, the cannula is formed of polyethylene, such as low density polyethylene.

Suitably, the cannula can be integrally formed.

The cannula may have a physiologically compatible lubricant coating applied to the outer surface of the elongate member prior to use, like for example petroleum jelly (Vaseline), mineral oil, or a water based lubricant. Alternatively, the cannula may comprise a lubricious coating such as a hydrophilic coating on its outer surface.

As outlined above, the cannula in some embodiments includes attachment means for attaching a reservoir thereto. The reservoir suitably includes an enema composition. In some embodiments, the enema composition is a liquid at room temperature and forms a gel at body temperature.

Suitably, the enema composition can include a therapeutic. In some embodiments, the enema composition is suitable for treating diseases or conditions of the rectum and/or colon.

The diseases of the rectum and/or colon may be selected from inflammatory bowel disease (IBD), inflammatory bowel syndrome (IBS), diverticulitis, small intestinal bacterial overgrowth (SIBO), ulcerative colitis, pouchitis, proctitis, proctosigmoiditis, left-sided colitis, pancolitis, fecal incontinence, ulcerative colitis, particularly distal ulcerative colitis, proctitis and proctosigmoiditis, or pouchitis. In an aspect, the enema composition is suitable for treating ulcerative colitis. In a further aspect, the enema composition is suitable for treating left-sided colitis (also known as distal ulcerative colitis). In a further aspect, the enema composition is suitable for treating proctosigmoiditis. In a further aspect, the enema composition is suitable for treating proctitis.

In some embodiments, the enema composition can include a therapeutic agent and a polymeric mixture.

In some embodiments, the polymeric mixture includes at least one thermo-responsive polymer (first polymer), at least one ion-sensitive polymer (second polymer) and at least one bio-adhesive polymer (third polymer). The mechanisms of action of these polymers are different. The thermo-responsive polymer (first polymer) gels or is able to create a structure into the body or body cavity when the temperature reaches a suitable range of values. The ion-sensitive polymer (second polymer) gels or is able to create a structure with the bio-relevant medium in the presence of specific ions in suitable concentrations. The bio-adhesive polymer (third polymer) provides improved adhesion of the enema composition and improved residence time at the target site. The bio-adhesive polymer (third polymer) ensures the improved adhesion through its interaction with the mucosa of the rectum, the colon, or in an artificial cavity surgically created (ileal pouch) (when treating pouchitis). Together, the bio-adhesive polymer enhances the grafting of the enema composition to the body tissue, whereas the first and second polymers enhance the structure of the gelling thin layer obtained by spreading the enema composition on the target body surface to act as a reservoir or a tridimensional matrix system to ensure the persistence and the prolonged pharmacological activity of a therapeutic agent in the gelled enema composition.

In some embodiments, the polymeric mixture includes at least one thermo-responsive polymer (first polymer), at least one ion-sensitive polymer (second polymer) and at least one bio-adhesive polymer (third polymer).

The first polymer may be selected from the group including but not limited to: polyoxyethylene-polyoxypropylene block copolymers, such as poloxamer 124, poloxamer 188, poloxamer 237, poloxamer 338, poloxamer 407 and the like, polyethylene glycol)/poly(lactide-coglicolide) block copolymers (PEG-PLGA), polyethylene glycol)-poly(lactic acid)-polyethylene glycol) (PEG-PLA-PEG), poly(N-isopropylacrylamide), and cellulose derivatives, like methylcellulose (MC) and hydroxypropylmethylcellulose (HPMC) and the like and mixtures thereof. In some embodiments, the first polymer is a polyoxyethylene-polyoxypropylene block copolymer selected from the group consisting of: poloxamer 124, poloxamer 188, poloxamer 237, poloxamer 338, and poloxamer 407; or a cellulose derivative selected from the group consisting of methylcellulose (MC), hydroxypropylmethylcellulose (HPMC) and mixtures thereof. In further embodiments, the first polymer is polyoxamer 188, poloxamer 407, or mixtures thereof.

The amount of the at least one thermo-responsive polymer (first polymer) can range between about 0.5% to about 30% by weight with respect to the weight of the (liquid) enema composition, in some embodiments between about 1% to about 25% by weight with respect to the weight of the (liquid) enema composition. In some embodiments, the at least one thermo-responsive polymer is present in an amount from about 5% to about 22% by weight with respect to the weight of the enema composition. In some embodiments, the at least one thermo-responsive polymer is present in an amount from about 10% to about 20% by weight with respect to the weight of the enema composition. In further embodiments the at least one thermo-responsive polymer is present in an amount between about 12% and about 18% by weight with respect to the weight of the (liquid) enema composition. Optimal in-situ gelification occurs when the at least one thermo-responsive polymer is present in an amount of from about 12 wt% to about 18 wt% with respect to the weight of the (liquid) enema composition, such as 12 wt%, or 13 wt%, or 14 wt%, or 15 wt%, or 16 wt%, or 17 wt%, or 18 wt%.

In some embodiments, said at least one thermo-responsive polymer is contained in an amount of about 0.5% or about 1.0% or about 5.0% or about 10.0% or about 14% or about 15% or about 16% or about 16.5% by weight with respect to the weight of the liquid enema composition.

The ion-sensitive polymer (second polymer) may be is a polysaccharide selected from the group including but not limited to: carrageenan, gellan gum, pectin, alginate, alginate salts and the like, alginic acid, and mixtures thereof. In some embodiments, the second polymer is sodium alginate.

The at least one ion-sensitive polymer may be present in an amount of from about 0.005% to about 5% by weight with respect to the weight of the (liquid) enema composition, in further embodiments from about 0.01% to about 3% by weight with respect to the weight of the (liquid) enema composition, in further embodiments from about 0.05% to about 2.5% by weight with respect to the weight of the (liquid) enema composition and in still further embodiments from about 0.1% to about 2.0% by weight with respect to the weight of the (liquid) enema composition. Optimal in-situ gelification occurs when the at least one ion-sensitive polymer is present in an amount of from about 0.1 wt% to about 2.0 wt% with respect to the weight of the (liquid) enema composition.

In some embodiments, the at least one ion-sensitive polymer is present in an amount of about 0.1% or about 0.2% or about 0.3%, or about 0.4% or about 0.5%, or about 0.6% or about 0.7%, or about 0.8%, or about 0.9%, or about 1%, or about 1.25%, or about 1.5%, or about 1.75%, or about 2% by weight with respect to the weight of the (liquid) enema composition.

The bio-adhesive polymer (third polymer) may be selected from the group including, but not limited to: chitosan, hyaluronic acid and salts thereof, cellulose derivatives, such as methyl cellulose, hydroxy propyl methylcellulose, hydroxy propyl cellulose, carboxymethyl cellulose sodium and the like, polyvinyl alcohol, polyvinyl pyrrolidone, polyacrylic acid, polyethylene oxides, cyclodextrins, tragacanth, sodium alginate, xanthan gum, gelatin, pectin, and the like and mixtures thereof. Any mixture of these bio-adhesive polymers may be used to obtain suitable enema compositions as defined herein. Suitably, the third polymer is a cellulose derivative selected from the group consisting of: methyl cellulose, hydroxy propyl methylcellulose, hydroxy propyl cellulose, carboxymethyl cellulose sodium and mixtures thereof.

In some embodiments, the at least one bio-adhesive polymer is carboxymethyl cellulose sodium, hyaluronic acid and/or a salt thereof, or mixtures thereof. The at least one bio-adhesive polymer (third polymer) provides an additional synergy with the first and second polymers leading to an improved level of adhesion of enema composition and an improved residence time at the desired site.

Suitably, the at least one bio-adhesive polymer is contained in an amount which ranges from about 0.005% to about 5% by weight with respect to the weight of the (liquid) enema composition. In some embodiments, the at least one bio-adhesive polymer is present in an amount of from about 0.02% to about 1.5% by weight with respect to the weight of the enema composition. In some embodiments, the at least one bio-adhesive polymer is present in an amount of from about 0.03% to about 1.0% by weight with respect to the weight of the enema composition. In some embodiments, the at least one bio-adhesive polymer is present in an amount of from about 0.04% to about 0.5% by weight with respect to the weight of the enema composition. In some embodiments, the at least one bio-adhesive polymer is present in an amount of 0.05% to about 0.1% by weight with respect to the weight of the (liquid) enema composition. Too much bio-adhesive polymer can lead to gelification prior to administration. Optimal in-situ gelification occurs when the bio-adhesive polymer is present in an amount of from about 0.05 wt% to about 0.1 wt% with respect to the weight of the (liquid) enema composition.

In some embodiments, the at least one bio-adhesive polymer is present in an amount of about 0.005% or about 0.01% and in some embodiments about 0.05% by weight with respect to the weight of the (liquid) enema composition.

Suitably, the at least one thermo-responsive polymer is present in an amount of from about 1% to about 25 % by weight with respect to the weight of the enema composition, optionally, in an amount from about 12% to about 18% by weight with respect to the weight of the enema composition.

Suitably, the at least one ion-sensitive polymer is present in an amount of from about 0.01% to about 3% by weight with respect to the weight of the enema composition, optionally, in an amount of from about 0.1% to about 2.0 % by weight with respect to the weight of the enema composition.

Suitably, the at least one bio-adhesive polymer is present in an amount of from about 0.01% to about 2.0% by weight with respect to the weight of the enema composition, optionally, in an amount of from about 0.05% to about 0.1% by weight with respect to the weight of the enema composition.

The enema composition may further includes at least one other excipient such as for example antioxidants, chelating agents, preservatives, antimicrobial agents, surfactants, co-surfactants, lipophilic compounds, purified water, organic salts, inorganic salts, buffers agents or mixtures thereof. In a some embodiment, the enema composition further includes at least one antioxidant and a least one preservative.

The enema composition may have a pH in the range of from 6 to 8, or in the range of from 6.5 to 7.5, or in the range of from pH 6.8 to 7.2.

In some embodiments, the first polymer gels or creates a structured state after contact with the bio-relevant medium or at physiological temperature (i.e., at about 37 °C); the action of the first polymer is further strengthened by the interaction of the second polymer with the target site, where the presence of specific trigger ions strengthens the interaction with the environmental medium of the other, resulting in an expansion of the polymer mixture's capability of forming a gel or a structured state. Upon contact with a site into the body or body cavity (for example, distal part of the gastro-intestinal tract, in the rectum or in an ileal pouch), characterized by a physiological temperature (i.e., ≥34°C), the third polymer further ensures and strengthens the bio-adhesion grafting the enema composition to the body tissue. The polymer mixture is effective because of an optimal mixture of the first, second and third polymers that lead to a synergism between the polymers themselves and the polymers with the microenvironment, leading to a strengthened gelation/structuring of the enema composition at the targeted site. Such strengthened gelation is demonstrated by a huge increase in the viscoelastic modulus G', as described in WO2019/122253.

In the preparation of the enema composition described herein, the choice of the suitable thermo-responsive polymers (first polymers) and of their concentration is made to obtain a final composition which is in liquid state below the body temperature (i.e. below about 37 °C, in some embodiments at about 20 °C to about 25 °C) and which becomes a gel, or a structured state once exposed to the body temperature or above (i.e., at or above about 37 °C). In the example composition, the therapeutic agent is rifamycin SV.

The cannula for rectal administration of an enema composition as defined herein is particularly effective for delivering enema compositions for treating ulcerative colitis, such as distal ulcerative colitis (also known as left-sided colitis), proctitis and/or proctosigmoiditis, or pouchitis.

Optionally, the enema composition further includes at least one therapeutic agent (or active substance) and at least one physiologically acceptable excipient.

Suitably, the therapeutic agent can be selected from the therapeutic classes comprising, but not limited to: aminosalycilates, corticosteroids, immunomodulators, tumor necrosis factor alpha (TNFα) inhibitors, integrin antagonists, interleukin antagonists, janus kinase (JAK) inhibitors, sphingosine 1 phosphate (S1p) modulators, oligonucleotides, antibiotics and probiotics.

In the therapeutic class of non-steroidal aminosalycilates, suitable therapeutic agents for the present disclosure provided can be selected in the group comprising: mesalamine (also known as mesalazine or 5-aminosalycilic acid), sulfasalazine, olsalazine, balsalazine, and the like.

In the therapeutic class of corticosteroids, suitable therapeutic agents for the present disclosure provided can be selected in the group comprising: betamethasone and salts thereof, hydrocortisone and salts thereof, budesonide, prednisone, prednisolone, methylprednisolone, beclomethasone and salts thereof, and the like.

In the therapeutic class of immunomodulators, suitable therapeutic agents for the present disclosure provided can be selected in the group comprising: azathioprine, cyclosporine A, 6-mercaptopurine, tacrolimus, and the like.

In the therapeutic class of tumor necrosis factor alpha (TNFα) inhibitors, suitable therapeutic agents for the present disclosure provided can be selected in the group comprising: infliximab, adalimumab, certolizumab pegol, golimumab and the like.

In the therapeutic class of integrin antagonists, suitable therapeutic agents for the present disclosure provided can be selected in the group comprising: natalizumab, vedolizumab and the like.

In the therapeutic class of interleukin antagonists, suitable therapeutic agents for the present disclosure provided can be selected in the group comprising: ustekinumab, risankizumab, mirikizumab and the like.

In the therapeutic class of janus kinase (JAK) inhibitors, suitable therapeutic agents for the present disclosure provided can be selected in the group comprising: tofacitinib, upadacitinib, filgotinib and the like.

In the therapeutic class of sphingosine 1 phosphate (S1p) modulators, suitable therapeutic agents for the present disclosure provided can be selected in the group comprising: ozanimod, estrasimod and the like.

In the therapeutic class of oligonucleotides, suitable therapeutic agents for the present disclosure provided can be selected in the group comprising: alicaforsen, cobitolimod and the like.

In the therapeutic class of antibiotics, suitable therapeutic agents for the present disclosure provided can be selected in the group comprising: penicillins (e.g., amoxicillin, ampicillin, dicloxacillin, oxacillin, piperacillin and the like), tetracyclines (democycline, doxycycline, eravacycline, minocycline, omadacycline, sarecycline, tetracycline and the like) cephalosporines (cefaclor, cefadroxil, cefdinir, cephalexin, cefprozil, cefdinir, cefepime, cefiderocol, cefotaxime, cefotetan, ceftaroline, ceftazidime, ceftriaxone, cefuroxime and the like), fluoroquinolones (ciprofloxacin, delafloxacin, levofloxacin, moxifloxacin, gemifloxacin and the like), lincomycines (clindamycin, lincomycin and the like), macrolides (azithromycin, clarithromycin, erythromycin, fidaxomicin and the like), sulfonamides (sulfamethoxazole, succinylsulfathiazole, sulfisoxazole and the like), glycopeptide antibiotics (dalbavancin, oritavancin, telavancin, vancomycin and the like), aminoglycosides (gentamicin, tobramycinm, amikacin and the like), carbapenems (imipenem, meropenem, ertapenem and the like), nitroimidazoles (metronidazole, tinidazole, nimorazole and the like), ansamycines (rifamycin SV or salts thereof, rifaximin, rifabutin, rifapentine, rifampicin and the like).

In some aspects, the enema composition contains more than one therapeutic agents, selected from the groups listed hereinabove.

In some aspects, the therapeutic agent is an aminosalicylate and is selected from mesalamine or olsalazine.

In other aspects, the therapeutic agent is a corticosteroid and is selected from busedonide or bethametasone or salts thereof.

In other aspects, the therapeutic agent is a TNFα inhibitor and is selected from infliximab or adalimumab.

In other aspects, the therapeutic agent is an integrin antagonist and is selected from natalizumab and vedolizumab.

In other aspects, the therapeutic agent is an interleukin antagonist and is selected from ustekinumab and mirikizumab.

In other aspects, the therapeutic agent is an S1p modulator and is selected from ozanimod and estrasimod.

In other aspects, the therapeutic agent is an olignucleotide and is alicaforsen.

In other aspects, the therapeutic agent is an antibiotic and is selected from ciprofloxacin, metronidazole, amoxicillin, vancomycin, rifaximin or rifamycin SV or salts thereof.

In some embodiments, the therapeutic agent is rifamycin SV or salts thereof.

In some embodiments, the therapeutic agent is comprised in the enema composition in an amount of about 0.001% to about 10%, from 0.01% to about 7.5%, or from 0.05% to about 5%. In some embodiments, the therapeutic agent is comprised in the enema composition in an amount of 1.0% or below, such as about 1.0%, or about 0.9%, or about 0.8% or about 0.7% or about 0.6% or about 0.5% or about 0.4% or about 0.3% or about 0.2% or about 0.1% or about 0.075% or about 0.5% or about 0.025% or about 0.01%. In other embodiments, the therapeutic agent is comprised in the enema composition in an amount between 1.0% and 2.0%, such as about 1.0%, or about 1.25%, or about 1.5%, or about 1.75%, or about 2.0%. In other embodiments, the therapeutic agent is comprised in the enema composition in an amount between 2.0% and 3.0%, such as about 2.0%, or about 2.25%, or about 2.5%, or about 2.75%, or about 3.0%. In other embodiments, the therapeutic agent is comprised in the enema composition in an amount between 3.0% and 4.0%, such as about 3.0%, or about 3.25%, or about 3.5%, or about 3.75%, or about 4.0%. In other embodiments, the therapeutic agent is comprised in the enema composition in an amount between 4.0% and 5.0%, such as about 4.0%, or about 4.25%, or about 4.5%, or about 4.75%, or about 5.0%. In other embodiments, the therapeutic agent is comprised in the enema composition in an amount between 5.0% and 6.0%, such as about 5.0%, or about 5.25%, or about 5.5%, or about 5.75%, or about 6.0%. In other embodiments, the therapeutic agent is comprised in the enema composition in an amount between 6.0% and 7.0%, such as about 6.0%, or about 6.25%, or about 6.5%, or about 6.75%, or about 7.0%. In other embodiments, the therapeutic agent is comprised in the enema composition in an amount between 7.0% and 8.0%, such as about 7.0%, or about 7.25%, or about 7.5%, or about 7.75%, or about 8.0%. In other embodiments, the therapeutic agent is comprised in the enema composition in an amount between 8.0% and 9.0%, such as about 8.0%, or about 8.25%, or about 8.5%, or about 8.75%, or about 9.0%. In other embodiments, the therapeutic agent is comprised in the enema composition in an amount between 9.0% and 10.0%, such as about 9.0%, or about 9.25%, or about 9.5%, or about 9.75%, or about 10.0%.Suitably, the enema composition for administration via the cannula described herein includes rifamycin SV, or a pharmaceutically acceptable salt thereof. In some embodiments, the enema composition includes a therapeutically effective amount of rifamycin SV, or a pharmaceutically acceptable salt thereof.

Suitably, the enema composition includes an active agent (rifamycin SV) and a polymeric mixture as described herein.

Rifamycin SV, being an ionic large molecule, is known to potentially interact with biopolymers, altering the respective properties (e.g., thermogelling, and/or bioadhesive and/or ionotropic properties). The enema compositions for rectal administration include a combination of the aforementioned three polymers, surprisingly allow to overcome this issue and to modulate the permanence time of said compositions at the target site.

Rifamycin SV is a semisynthetic antibacterial belonging to the ansamycin class; it exerts its antibacterial activity by irreversibly binding to the bacterial DNA-dependent RNA polymerase β-subunit, thereby inhibiting bacterial RNA synthesis. Rifamycin SV exhibits an antibacterial activity against most bacterial enteropathogens frequently associated with infectious diarrhea and other enteric infections, including *Enterobacteriaceae* and non*-Enterobacteriaceae* [Clin Drug Investig 2019 Jul., 39(7):691-697]. Rifamycin SV corresponds to the sodium salt of Rifamycin or Rifamycin sodium. For colonic diseases like ulcerative colitis, bacterial proliferation or microbial dysbiosis is associated with a strong inflammatory component. This inflammation has a profound influence on the liver via the gut-liver axis. Rifamycin SV is endowed with anti-inflammatory activities based on the impact on two key regulators of inflammation: pregnane X receptor (PXR) and nuclear factor- κB (NFκB). Rifamycin SV was found to activate PXR and two of its downstream targets, cytochrome P450 3A4 (CYP3A4) and P-glycoprotein (PgP), in liver and intestinal cell lines. Rifamycin also directly inhibited NFκB in a cell line which lacks PXR expression [Rosette, et al., 2019]. These dual activities likely explain the inhibition of pro-inflammatory cytokine secretion from human colonic cells lines and activated CD4+ T cells [Rosette, et al., 2013]. Rifamycin exerts its clinical benefit as topical action into the intestinal tract and it is characterized by a negligible absorption in the bloodstream. Rifamycin is a safe drug and has a favorable safety profile as compared to for example ciprofloxacin and metronidazole currently used in the treatment of gastrointestinal diseases with an inflammatory component, such as ulcerative colitis (including proctitis and proctosigmoiditis).

In some embodiments, the active agent present in the enema composition is Rifamycin SV or pharmaceutically acceptable salts thereof. The Rifamycin SV or pharmaceutically acceptable salts thereof may be present in an amount which ranges from about 0.01% to about 10% by weight with respect to the weight of the (liquid) enema composition, in some embodiments from about 0.1% to about 5% by weight with respect to the weight of the (liquid) enema composition, and in further embodiments from about 0.25% to about 3% by weight with respect to the weight of the (liquid) enema composition, such as from about 0.25% to about 2.5% by weight with respect to the weight of the (liquid) enema composition. In some embodiments, Rifamycin SV or pharmaceutically acceptable salts thereof is contained in an amount of about 0.1%, or 0.2%, or 0.3%, or 0.4%, or 0.5%, or 0.6%, or 0.7%, or 0.8%, or 0.9%, or 1.0%, or 1.1%, or 1.2%, or 1.3%, or 1.4%, or 1.5%, or 1.6%, or 1.7% or 1.8% or 1.9% or 2.0% with respect to the weight of the (liquid) enema composition. In some embodiments, Rifamycin SV or pharmaceutically acceptable salts thereof is contained in an amount of about 0.1% or about 0.2 % or about 0.25% or about 0.3% or about 0.4% or about 0.5% or about 1% or 2.5% and in further embodiments about 0.5% or 1.0% by weight with respect to the weight of the enema composition.

Rifamycin SV or pharmaceutically acceptable salts thereof may be present in an amount of about 0.5% by weight with respect to the weight of the (liquid) enema composition.

Rifamycin SV or pharmaceutically acceptable salts thereof may be present in an amount of about 1.0% by weight with respect to the weight of the (liquid) enema composition.

In some embodiments, the dosage strength of Rifamycin SV or pharmaceutically acceptable salts thereof in an enema composition of the present disclosure is established in order to achieve a local concentration of Rifamycin SV in the site of action exceeding its Minimum Inhibitory Concentration (MIC) on the specific bacterial population commonly localized therein. The MIC is defined as the lowest concentration expressed in mg/L (equivalent to µg/mL) of an antimicrobial or antibacterial active agent that is bacteriostatic, i.e., prevents the visible growth of bacteria. MICs are used to evaluate the antimicrobial efficacy of various compounds by measuring the effect of decreasing concentrations of antibiotic over a defined period in terms of inhibition of microbial population growth. MIC is generally regarded as the most basic laboratory measurement of the activity of an antimicrobial agent against an organism. Because a lower MIC value indicates that less of the drug is required in order to inhibit growth of the organism, drugs with lower MIC values are more effective antimicrobial or antibacterial active agents.

The enema composition may be prepared to contain a dosage of Rifamycin SV and stored for future use. In another embodiment, the enema composition could be prepared without the active agent and could be stored until usage. A suitable dosage of rifamycin SV, as described herein above, can thereafter be added to such enema composition prior to administration.

Rifamycin SV or pharmaceutically acceptable salts thereof may be fully dissolved in an enema composition of the present disclosure. Rifamycin SV or pharmaceutically acceptable salts thereof may be partly dissolved and partly in solid suspension in the enema composition of the present disclosure. The enema composition in some embodiments contains one or more pharmaceutically acceptable excipients.

The pharmaceutically acceptable excipients may be selected in the group including: diluents, stabilizers, preservatives, antioxidants, buffers and other ingredients that are appropriate to the nature, composition and mode of administration of the enema composition described herein. The types and amounts of pharmaceutically acceptable excipients that can be used in the enema composition can be readily determined by the skilled artisan. The enema composition may contain at least one anti-oxidant as a pharmaceutically acceptable excipient. The enema composition containing the at least one anti-oxidant may further includes at least one preservative as a further pharmaceutically acceptable excipient. Anti-oxidants and preservatives which are pharmaceutically acceptable are well known to the skilled artisan.

The enema composition may includes water or liquid carrier, or a physiologically acceptable solvent and optionally an alcohol, such as ethanol. For example, the liquid carrier may be water or a physiologically acceptable solvent or a mixture of water and one or more physiologically acceptable solvents. Typical such solvents include, for example, glycerol, ethylene glycol, propylene glycol, polyethylene glycol and polypropylene glycol. The liquid carrier can be, for example, water.

In the preparation of the enema composition described herein including the polymer mixture, the choice of the suitable ion-sensitive polymer (second polymer) and of their concentration is made to obtain a suitable sol-gel transition or a transition to a structured composition in the presence of specific ions.

As described herein, an ion-sensitive polymer can be selected among those able to bind mono and/or divalent inorganic ions, such as Na⁺, K⁺, Mg²⁺, Ca²⁺ and Zn²⁺, and organic ions with high affinity. The responsiveness to ionic strength is a typical property of polymers containing ionizable groups. Changes in ionic strength may cause changes in the size of the polymeric micelles and polymer solubility.

Also disclosed herein is a kit of parts including:
(i) a first package enclosing a cannula as defined herein, and
(ii) a second package encasing a reservoir including an enema composition.

According to a further aspect, there is provided a kit of parts comprising:
(i) a first package enclosing a cannula as defined herein, and
(ii) a second gas impermeable package encasing a reservoir containing an enema composition, wherein optionally, the second package is an aluminum pouch.

According to a further aspect, there is provided a kit of parts comprising:
(i) a first package enclosing a cannula as defined herein, and
(ii) a second gas impermeable package encasing: (a) a reservoir including an enema composition, and (b) an oxygen scavenger, wherein optionally, the second package is an aluminum pouch.

Suitably, the package enclosing the cannula is plastic.

Optionally, the cannula in the package can be sterilized. For example, by exposure to radiation.

Suitably, the reservoir is a bottle, such as a bellows bottle, optionally, the bottle is sealed with a cap. Optionally, the cap is a screw cap.

Suitably, the reservoir has a volume between 10 mL and 200 mL, between 30 mL and 150 mL, or between 50 mL and 120 mL. In some embodiments, the reservoir has a volume of 30 mL, or 40 mL, or 50 mL, or 60 mL, or 70 mL, or 80 mL, or 90 mL, or 100 mL, or 110 mL, or 120 mL.

Suitably, the second package is an aluminum pouch, in some embodiments, a hermetically sealed aluminum pouch.

Optionally, the oxygen scavenger is added to the aluminum pouch.

Disclosed herein is a method of treating, ameliorating, reducing the severity of, slowing the progression of, inducing the remission of, or maintaining the remission of ulcerative colitis, such as distal ulcerative colitis (also known as left-sided colitis), ulcerative proctitis and/or proctosigmoiditis), including employing a cannula as defined herein to administer an enema composition to a patient in need thereof, wherein the enema composition is a liquid composition which forms a gel at body temperature.

According to a further aspect of the present disclosure, there is a provided a method of treating, ameliorating, reducing the severity of, slowing the progression of, inducing the remission of, or maintaining the remission of pouchitis, including employing a cannula as defined herein to rectally administer an enema composition to a patient in need thereof, wherein the enema composition is a liquid composition at room temperature and forms a gel at body temperature.

In some embodiments, the liquid enema composition includes a therapeutically effective amount of rifamycin SV, or a pharmaceutically acceptable salt thereof.

As described herein, the induction of remission and the maintenance of remission must be intended as including, but not limited to, the alleviation or the amelioration or the reduction of the clinical symptoms, endoscopic symptoms or the histologic symptoms or a combination thereof. In some embodiments, the administration of the enema composition alleviates and/or reduces and/or ameliorates one or more clinical symptoms selected from a group including, but not limited to: increased frequency of evacuations (also referred to as change in bowel habit), change in fecal consistency, rectorrhagia, hematochezia, pain, cramp-like abdominals, urgency, tenesmus, fecal incontinence, fever and/or extraintestinal manifestations, or combination thereof.

For the purpose of the present disclosure, the induction of remission and the maintenance of remission may be including, but not limited to, the alleviation or the amelioration or the reduction of the clinical symptoms, endoscopic symptoms or the histologic symptoms or a combination thereof. In some embodiments, the administration of the enema composition using the rectal cannula described herein alleviates and/or reduces and/or ameliorates one or more clinical symptoms selected from a group including, but not limited to: increased frequency of evacuations (also referred to as change in bowel habit), change in fecal consistency, rectorrhagia, hematochezia, pain, cramp-like abdominals, urgency, tenesmus, fecal incontinence, fever and/or extraintestinal manifestations, or combination thereof. In some embodiments, the administration of the enema composition using the rectal cannula described herein alleviates and/or reduces and/or ameliorates the occurrence of one or more endoscopy symptoms selected from the group including, but not limited to: mucosal edema, granularity, contact bleeding, loss of vascular pattern, hemorrhage, and ulceration. In an embodiment, the administration of the enema composition alleviates and/or reduces and/or ameliorates the occurrence of one or more histologic symptoms selected from the group including, but not limited to: polymorph infiltration, ulceration, erosions, mononuclear leukocytes infiltration, and villous atrophy.

The treatment regimen and administration schedule of the enema composition of the present disclosure to subjects in need thereof may be adapted, depending upon the nature of the disease (e.g., acute or chronic), its severity and the overall condition of the patient. In some embodiments, the treatment period with the composition of the present disclosure may last for about 2 weeks to about 4 weeks for each cycle of treatment. In some embodiments, the treatment period with the composition of the present disclosure may last for about 1 week, 2 weeks, 3 weeks, or 4 weeks. In some embodiments, the treatment period with the composition of the present disclosure may last 2 weeks. In further embodiments, the treatment period with the composition of the present disclosure may last 4 weeks.

In some embodiments, the treatment period with the composition of the present disclosure may last for about 2 weeks to about 8 weeks for each cycle of treatment. In some embodiments, the treatment period with the composition of the present disclosure may last for about 2 weeks, 3 weeks, 4 weeks, 5 weeks, 6 weeks, 7 weeks or 8 weeks. In some embodiments, the treatment period with the composition of the present disclosure may last 4 weeks. In further embodiments, the treatment period with the composition of the present disclosure may last 6 weeks. In further embodiments, the treatment period with the composition of the present disclosure may last 8 weeks.

In some embodiments, the enema composition may be administered using the rectal cannula described herein once daily, or twice a day, or three times a day, or four times a day. In some embodiments, the enema composition of the present disclosure is administered once daily. In further embodiments, the enema composition of the present disclosure is administered twice a day.

In some embodiments, the enema composition is self-administered by the patient in need thereof using the rectal cannula described herein.

In some embodiments, an enema composition including 0.5% by weight of Rifamycin SV may be administered rectally using the cannula described herein once daily, in a single dose of 60 mL enema.

In some embodiments, an enema composition including 1.0 % by weight of Rifamycin SV may be administered rectally using the cannula described herein once daily, in a single dose of 60 mL enema.

In some embodiments, an enema composition including 0.5% by weight of Rifamycin SV may be administered rectally using the cannula described herein twice a day, in a single dose of 60 mL enema.

In some embodiments, an enema composition including 1.0% by weight of Rifamycin SV may be administered rectally using the cannula described herein twice a day, in a single dose of 60 mL enema.

In some embodiments, an enema composition including 0.5% by weight of Rifamycin SV may be administered rectally using the cannula described herein once daily, in a single dose of 80 mL enema.

In some embodiments, an enema composition including 1.0 % by weight of Rifamycin SV may be administered rectally using the cannula described herein once daily, in a single dose of 80 mL enema.

In some embodiments, an enema composition including 0.5% by weight of Rifamycin SV may be administered rectally using the cannula described herein twice a day, in a single dose of 80 mL enema.

In some embodiments, an enema composition including 1.0% by weight of Rifamycin SV may be administered rectally using the cannula described herein twice a day, in a single dose 80 mL enema.

In some embodiments, an enema composition including 0.5% by weight of Rifamycin SV may be administered rectally using the cannula described herein once daily, in a single dose of 100 mL enema.

In some embodiments, an enema composition including 1.0 % by weight of Rifamycin SV may be administered rectally using the cannula described herein once daily, in a single dose of 100 mL enema.

In some embodiments, an enema composition including 0.5% by weight of Rifamycin SV may be administered rectally using the cannula described herein twice a day, in a single dose of 100 mL enema.

In some embodiments, an enema composition of the present disclosure including 1.0% by weight of Rifamycin SV may be administered rectally using the cannula described herein twice a day, in a single dose of 100 mL enema.

In some embodiments, the treatment with the enema composition using the rectal cannula described herein can be done once, to achieve the induction of remission, or maintain the remission of the disease in a subject in need thereof. In other embodiments, the treatment with the enema compositions using the rectal cannula described herein can be repeated cyclically, to maintain the remission of the disease, and/or prevent the relapse of the disease, in a subject in need thereof. According to the latter embodiments, such cyclic treatments with the enema composition of the present disclosure can be repeated once per month or every two months, or every three months, or every four months, or every five months, or every six months. According to some embodiments, such cyclic treatments with the enema composition of the present disclosure is repeated once per month. According to some embodiments, such cyclic treatments with the enema composition of the present disclosure is repeated once every two months. According to further embodiments, such cyclic treatments with the enema composition of the present disclosure is repeated once every three months. The frequency of the cycles of treatments with the enema composition of the present disclosure depend upon the frequency of relapse of the disease. The frequency of the cycles of treatments with the enema composition of the present disclosure depend upon the maintenance of the remission. In some embodiments, the frequency of the treatments in case of maintenance of the remission is each month from two weeks to four weeks.

In some embodiments, the enema composition described herein can be used to deliver Rifamycin SV directly in the targeted rectosigmoid region of the large intestine by direct, topical administration. In some embodiments, the enema composition is suitable for rectal administration. In some embodiments, the enema composition is in the form of an enema. In some embodiments, the enema composition is a liquid in the form of a solution, suspension, foam, emulsion or microemulsion; in some embodiments the enema composition is in form of a solution and in further embodiments an aqueous solution.

In some embodiments, the enema composition of the disclosure is in the form of an enema and is contained in a reservoir (such as a bottle, such as, for example, a bellows enema bottle) whose individual content per unit is in the range between 10 mL and 200 mL, between 30 mL and 150 mL, or between 50 mL and 120 mL. In some embodiments, the individual content per unit of the reservoir containing the enema compositions of the disclosure in form of enema is 50 mL, or 60 mL, or 70 mL, or 80 mL, or 90 mL, or 100 mL, or 110 mL, or 120 mL, or 130 mL, or 140 mL, or 150 mL. In some embodiments, the individual content per unit of the reservoir containing the enema compositions of the disclosure in the form of an enema is 50 mL. In further embodiments, the individual content per unit of the enema compositions of the disclosure in form of enema is 60 mL. In further embodiments, the individual content per unit of the reservoir containing the enema compositions of the disclosure in form of enema is 80 mL. In further embodiments, the individual content per unit of the reservoir containing the enema compositions of the disclosure in form of enema is 100 mL.

Some enema compositions of the present disclosure can be used for the treatment of diseases affecting the distal part of the large intestine, more specifically the sigmoid colon and rectum. In some embodiments, such diseases affecting the distal part of the large intestine are proctitis and/or distal ulcerative colitis and/or proctosigmoiditis. The enema composition is prepared by serially adding the components of the enema composition to water with stirring until complete dissolution or a homogeneous mixture is obtained eventually having the Rifamycin SV partially suspended as more fully described in the Examples below.

In some embodiments, the enema composition includes rifamycin SV as the active agent and a polymer mixture including at least one thermo-responsive polymer, at least one ion-sensitive polymer and at least one bio-adhesive polymer. In some embodiments, the at least one thermo-responsive polymer may include poloxamer 407, the at least one ion-sensitive polymer may include alginic acid or salts thereof, and the at least one bio-adhesive polymer may include sodium carboxymethylcellulose. In some embodiments, the polymer mixture includes poloxamer 407, sodium alginate and sodium carboxymethylcellulose.

In some embodiments, poloxamer 407 may be present in an amount of about 5 % to about 30% by weight with respect to the weight of the enema composition, optionally in the range of about 10% to about 25%, with respect to the weight of the enema composition or in the range of about 12% to about 18% with respect to the weight of the enema composition, and in some embodiments about 10%, or 11%, or 12%, or 13%, or 14%, or 15%, or 16% or 17% or 18% by weight with respect to the weight of the enema composition. In some embodiments, poloxamer 407 may be present in an amount of about 16.5% by weight with respect to the weight of the enema composition.

In some embodiments, sodium alginate may be present in an amount of about 0.005% to about 5% by weight with respect to the weight of the enema composition, optionally in the range of about 0.001% to about 3.0% by weight with respect to the weight of the enema composition, or in the range of about 0.05% to about 2.0% by weight with respect to the weight of the enema composition, in some embodiments 0.01%, or 0.02%, or 0.03%, or 0.04%, 0.05%, or 0.06%, or 0.07%, or 0.08%, or 0.09%, or 0.1% (w/w), or 0.15%, or 0.2%, or 0.25%, or 0.3%, or 0.35%, or 0.4%, or 0.45%, or 0.5%, or 0.55%, or 0.6%, or 0.65%, or 0.7%, or 0.75%, or 0.8%, or 0.85%, or 0.9%, or 0.95%, or 1.0% with respect to the weight of the enema composition.

In some embodiments, sodium carboxymethylcellulose may be present in an amount of about 0.005% to about 5% by weight with respect to the weight of the enema composition, optionally in the range of about 0.001% to 2% by weight with respect to the weight of the enema composition, or in the range of about 0.05% to about 0.1% by weight with respect to the weight of the enema composition, in some embodiments about 0.01%, or 0.02%, or 0.03%, or 0.04%, 0.05%, or 0.06%, or 0.07%, or 0.08%, or 0.09%, or 0.1% (w/w), or 0.15%, or 0.2%, or 0.25%, or 0.3%, or 0.35%, or 0.4%, or 0.45%, or 0.5%, or 0.55%, or 0.6%, or 0.65%, or 0.7%, or 0.75%, or 0.8%, or 0.85%, or 0.9%, or 0.95%, or 1.0% with respect to the weight of the composition.

In some embodiments, the enema composition may further contain at least one anti-oxidant. In some embodiments, the anti-oxidant may be potassium metabisulfite, ascorbic acid, or sodium ascorbate or mixtures thereof. In some embodiments, the anti-oxidant includes these three anti-oxidants.
In some embodiments, the enema composition may further contain at least one preservative. In some embodiments, the preservative may be potassium sorbate, sodium benzoate or ethanol or mixtures thereof. In some embodiments, the preservative includes ethanol or a mixture of potassium sorbate and sodium benzoate.

In some embodiments the mixture of the polymers and the Rifamycin SV is a unique system in which the amount of the Rifamycin SV in soluble form as well as partially suspended form influences the rheological properties of the mixture of polymers. The rheological properties unexpectedly decrease with an increase of the amount of Rifamycin SV added to the enema composition. In some embodiments, the enema composition containing 0.25 to 3.0% by weight of Rifamycin SV has a qualitative/quantitative composition of the selected polymers which leads the starting of sol to gel transition at a temperature in between 27°C and 37°C. For example, the enema composition containing 0.5% by weight or 1.0% by weight of Rifamycin SV has a qualitative/quantitative composition of the selected polymers which leads the starting of sol to gel transition at a temperature in between 27°C and 37°C. For example, the enema composition containing 0.5% by weight or 1.0% by weight of Rifamycin SV gelifies at body temperature (around 37°C) and in turn this allows to manage the product at room temperature as liquid form (patient's compliance) and to have a gel product at the body temperature (around 37°C) after administration by rectal route. The addition of Rifamycin SV into the polymer mixtures provides the improved long-lasting effect of the gel overtime at the target site compared with the polymers mixture alone. Unexpectedly the Rifamycin SV interacts with the polymers mixture increasing the G' modulus overtime allowing the Rifamycin SV to remain embedded in the thin layer of the gel in tight contact with the site of action, and prolonging the duration of its pharmacological activity in situ strengthening the gel structure at the target site. As such, the enema compositions of the present disclosure, formulated as in situ gelling enema containing Rifamycin SV, exert an anti-inflammatory activity which is higher than the Rifamycin SV alone, and are of particular utility in treating, ameliorating, reducing the severity of, inducing, maintaining the remission of, and/or slowing the progression of gastrointestinal diseases with an inflammatory component, such as proctitis, ulcerative colitis, and proctosigmoiditis.

The enema composition of the disclosure, including Rifamycin SV and the three polymers as above disclosed, is thus able to provide a long-lasting effect and enhanced adhesion of Rifamycin SV or pharmaceutically active salts thereof at the site of action (distal part of the gastro-intestinal tract and/or the artificial cavity surgically created ileal pouch) when administered using the rectal cannula described herein, as the combination of the rectal cannula and the enema composition properties give rise to improved distribution, increased coverage and reduced waste, avoiding the quick evacuation of the composition with the significant loss of activity. Moreover, Rifamycin SV showed a dual therapeutic effect, combining an antibiotic action with an anti-inflammatory effect, providing a complete therapeutic system with a broad spectrum of action. Up to date there is no treatment able to combine a dual therapeutic effect together with a higher permanence time at the site with an improved long-lasting adhesion.

The anti-inflammatory efficacy, together with the antibiotic effect of the Rifamycin SV formulated in the enema composition of the disclosure including the three polymer mixtures leads to an effect in treating, ameliorating, reducing the severity of, inducing, maintaining the remission of, and/or slowing the progression of gastrointestinal diseases with an inflammatory and/or infectious component. Rifamycin's short-term but immediate clinical benefit of reducing inflammation, its clinical benefit of bacterial infection eradication, and minimal absorption at the intestinal level, renders the composition of the disclosure advantageous treatments for gastrointestinal diseases with an inflammatory and/or infectious component.

In the treatment of distal ulcerative colitis (including proctosigmoiditis) and/or proctitis, the formulations according to the present disclosure offer substantial advantages over the currently available treatments. As matter of fact, the enema compositions of the disclosure were demonstrated to undergo a sol to gel transition in physiological conditions, allowing for a prolonged retaining of the compositions themselves at the site of action upon administration via the rectal cannula described herein, whereas the currently available treatments remain liquid (enemas or rectal foams) or liquefy at body temperature (suppositories), and are therefore expelled from the body in a short time due to their physical structure. Moreover, the compositions of the present disclosure offer the substantial advantage of targeting two components that are known to be involved in the pathogenesis of such diseases, i.e. the intestinal bacteria, which are responsible for the activation of the innate immune system, and the consequent inflammation. The currently available treatments only target the inflammation.

Another advantage of the enema compositions of the present disclosure is constituted by an improved stability of solutions of rifamycin and salts thereof. As further detailed in the Examples, the enema compositions of the disclosures were placed in stability studies under different storage conditions, along with a commercially available liquid formulation of rifamycin (Rifocin^{®}). The results of the analyses show that the rifamycin active substance shows a less degree of degradation as compared to a standard liquid formulation of rifamycin. The increased stability simplifies the handling of the product, allowing a storage at room temperatures for a period of at least some months.

### DETAILED DESCRIPTION OF FIGURES

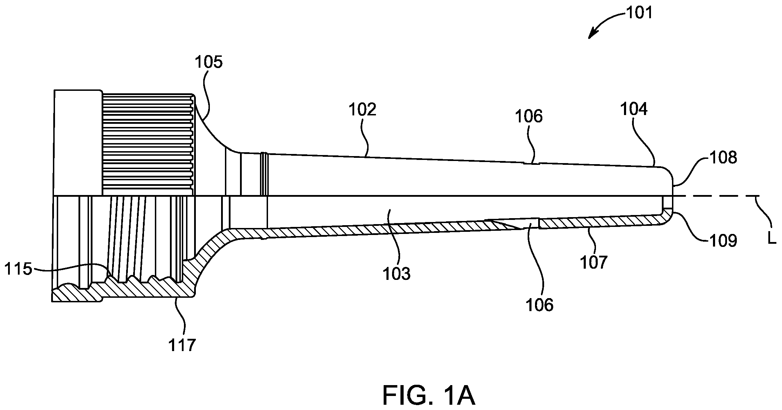

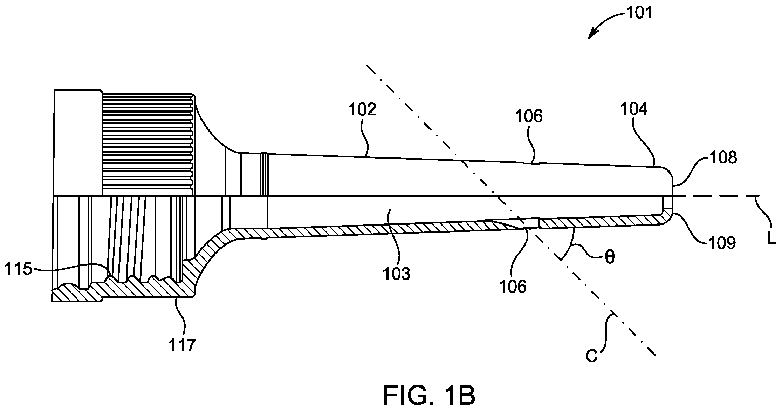

FIG. 1A shows a cannula (101) for rectal administration of an enema including: an elongate member (102) having a lumen (103), said elongate member (102) having a proximal insertion end (104) and a distal end (105), and at least one lateral dispensing opening (106) extending through a sidewall (107) of the elongate member (102) and configured to dispense fluid from the lumen. In some embodiments, the at least one lateral dispensing opening (106) can be configured to dispense fluid from the lumen at an angle of from about 30° to about 60° to a longitudinal axis (L) of the elongate member. By dispensing an enema composition at an angle of from about 30° to about 60° to a longitudinal axis (L) of the elongate member distribution of the enema within the rectum and colon can be improved. Thus, the cannula in some embodiments of the present disclosure has a dispensing angle from the lateral dispensing openings of from about 30° to about 60° to a longitudinal axis (L) of the elongate member application. This dispensing angle can advantageously significantly increase the application contact surface area of the composition, particularly in the rectum, in comparison to when an enema composition is administered rectally via a cannula including only an apical dispensing opening. Furthermore, the application contact surface area is also significantly increased when using the cannula of the present disclosure having a dispensing angle in the range of from about 30° to about 60° to a longitudinal axis (L) of the elongate member, in comparison to the corresponding contact surface area when a cannula having a dispensing angle that is orthogonal (or substantially orthogonal) to a longitudinal axis of the cannula shaft is used. FIG. 1B shows a dispensing opening (106) defined in a sidewall of the elongate member having a central axis (C) extending at an angle θ of approximately 45° to the longitudinal axis (L) of the elongate member.

In FIG. 1A and FIG. 1B an apical dispensing opening (108) is defined in a tip (109) of the proximal insertion end (104). The presence of the apical dispensing opening (108) ensures the enema composition is administered into the upper regions of the rectum, past the rectosigmoid junction and into the sigmoid colon. In some embodiments, including the embodiments illustrated in FIGS. 1A and 1B as well as FIGS. 2 and 3A and 3B, the cannula (101) can include two lateral dispensing openings (106) as described above. Two lateral dispensing openings (106) as shown are sufficient to provide adequate coverage with the enema compositions disclosed herein around an entire circumference of the cannula (101) i.e. circumferential delivery of the enema composition from the cannula. Thus, the cannula (101) according to embodiments including those illustrated in FIGS. 1A, B, 2, 3A and 3B may be used without regard to its angular position about the longitudinal axis L relative to the patient. Moreover, the use of two lateral dispensing openings (106) having dimensions in approximately the illustrated proportions to other features of the cannula (101) as described herein enables adequate lateral dispensation of the enema composition without undue loss of pressure for solution dispensed from the apical dispensing opening (108). Thus, in such embodiments, the addition of lateral dispensing openings (106) does not impair the cannula's (101) ability to deliver adequate amounts of enema composition to the sigmoid colon through the apical dispensing opening (108). Advantageously, approximately 20 to 50 wt% of the enema composition, such as 30 to 50 wt% of the enema composition may be administered via the lateral dispensing openings (106) of the cannula.

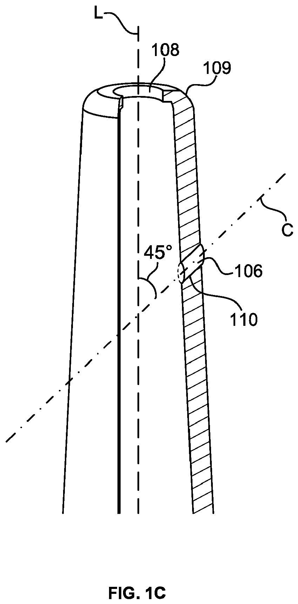

FIG. 1C is a partial cut-away section of a cannula of the present disclosure wherein illustrating the three dimensional character of the dispensing opening, and in the embodiment shown in FIG. 1C, the dispensing conduit (110) is circular in cross sectional area when viewed parallel to the longitudinal axis L of the elongate member. FIG. 1C shows a dispensing opening (106) defined in a sidewall of the elongate member having a central axis (C) extending at an angle θ of approximately 45° to the longitudinal axis (L) of the elongate member.

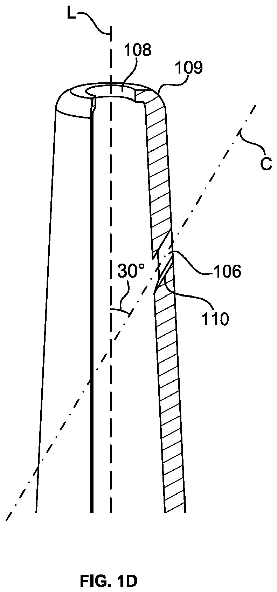

FIG 1D, is a partial cut-away section of a cannula of the present disclosure where the dispensing opening is defined in the sidewall having a rectangular cross section when viewed parallel to the longitudinal axis L of the elongate member. FIG. 1D shows a dispensing opening (106) defined in a sidewall of the elongate member having a central axis (C) extending at an angle θ of approximately 30° to the longitudinal axis (L) of the elongate member.

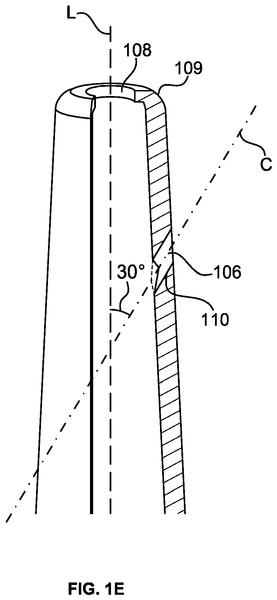

FIG. 1E is a partial cut-away section of a cannula illustrating of the present disclosure where the dispensing opening is defined in the sidewall having a diamond cross-section when viewed parallel to the longitudinal axis L of the elongate member.

Each cannula shown in FIG. 1C to 1E is shown with a single lateral dispensing opening (106). Such embodiments may comprise two or more lateral dispensing openings, which may or may not be offset with respect to the longitudinal axis L of the elongate member (102).

As shown in FIG. 1A and FIG. 1B, the cannula (101) can include attachment means to facilitate attachment of the cannula (101) to a reservoir for the enema composition. In some embodiments, such means can include a cuff (117) with threading (115). The cuff (117) may be located at the distal end (105) of the elongate member (102). The threading (115) may be configured to threadedly engage with complementary threading on a feature of the reservoir. In some embodiments, the threading (115) can be internal threading within the cuff (117). However, in further embodiments, the threading (115) can be external threading. In further embodiments, the attachment means of the cannula (101) can be features other than a threaded cuff, and the reservoir can include other complementary features configured to engage the other attachment means.

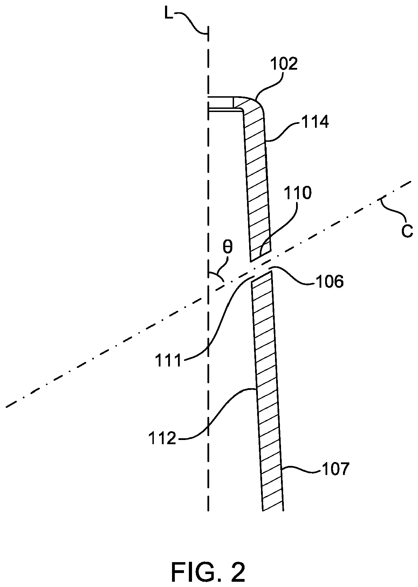

FIG. 2 shows a partial cross-section along the longitudinal axis L of the elongate member. The lateral dispensing opening (106) is defined by a (lateral) dispensing conduit (110) in the sidewall (107) of the elongate member (102), said dispensing conduit (110) extending from an internal opening (111) in an inner surface (112) of the elongate member (102) and an external opening (113) in an outer surface (114) of the elongate member (102), said dispensing conduit (110) including a central axis (C) extending at an angle θ of from about 30° to about 60° to a longitudinal axis (L) of the elongate member (102). In the embodiment shown in FIG. 2, the dispensing conduit (110) of the dispensing opening (106) is defined at an angle θ of 60° to the longitudinal axis L of the elongate member (102).

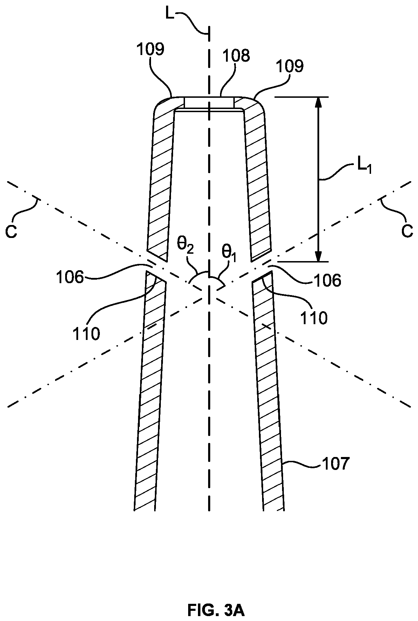

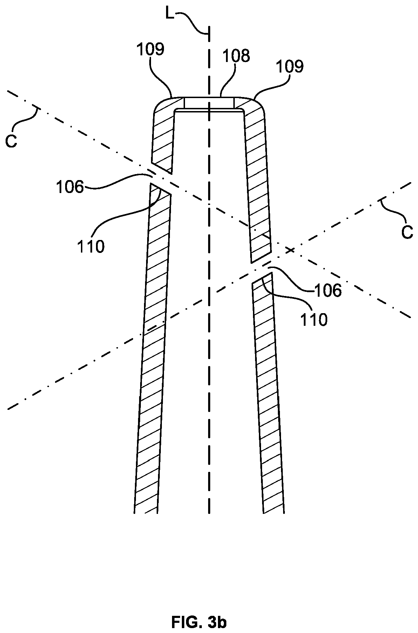

FIG. 3A shows a partial cross-section through the elongate member (102). The embodiment in FIG. 3A includes two lateral dispensing openings (106) and one apical dispensing opening (108). The lateral dispensing openings (106) are defined in the sidewall of the elongate member at a length l₁ from the tip (109) of the proximal insertion end (104). In some embodiments, at least one lateral dispensing opening (106) is disposed approximately 0.5 to 2.5 cm from the tip of the proximal insertion end, in some embodiments, at least 1 cm to 2 cm from the tip of the proximal insertion end. In the embodiment of FIG. 3a, the lateral dispensing openings are approximately 1.5 cm from the tip of the proximal insertion end. In the embodiment shown in FIG. 3a the dispensing conduit (110) of each lateral dispensing opening (106) is defined at an angle θ of 60° to the longitudinal axis L of the elongate member (102). FIG. 3B shows a further cannula of the present disclosure comprising two lateral dispensing openings (106) that are offset along the longitudinal axis L of the elongate member.

In the embodiments shown in FIG. 2 and FIG. 3A the circumference of the external opening in the outer surface is approximately the same length as the circumference of the internal opening, however, in the embodiment shown in FIGS. 1A and 1B, the circumference of the external opening is shorter in length than the circumference of the internal opening. This causes the dispensing conduit to form a nozzle through the sidewall extending from the lumen. In the embodiment shown in FIGS. 1A and 1B, the nozzle is a converging nozzle. In some embodiments, including those illustrated in FIGS. 1A and 1B, the lateral dispensing openings (106) taper to be longitudinally narrower with increasing distance from the longitudinal axis (L) and proximity to the exterior surface of the elongate member (102). In such embodiments, the lateral dispensing openings (106) themselves may act as converging nozzles that cause enema composition to exit the elongate member (102) at relatively high speed and low turbulence compared to openings with similar, but constant, cross-sectional area. In further embodiments, including some embodiments with converging lateral dispensing openings (106) as shown in FIGS. 1A and 1B as well as some embodiments with lateral dispensing openings (106) as shown in FIGS. 2 and 3A and 3B, the lateral dispensing openings (106) may each be centered on a central axis (C). In some embodiments, the central axis (C) may extend at an angle θ that is less than 90° from the longitudinal axis (L). In some embodiments, the angle θ of less than 90° may be within a range from about 30° to about 60° to a longitudinal axis (L) as described above. In further embodiments, the angle θ may be less than 90° on a proximal side of the intersection between the central axis (C) and the longitudinal axis (L). Thus, by extending along a central axis (C) forming a proximal-side angle θ of less than 90° from the longitudinal axis (L), each lateral dispensing opening (106) may be inclined proximally, meaning toward the proximal insertion end (104) of the elongate member (102), with increasing distance from the longitudinal axis (L). Such proximally inclined lateral dispensing openings (106) inclined this way may impart a proximal component to the velocity of enema composition dispensed through the lateral dispensing openings (106), meaning the enema composition dispensed through the lateral dispensing openings (106) may be directed deeper into the colon as well as laterally from the elongate member (102). Expelling the enema composition dispensed through the lateral dispensing openings (106) in a partially proximal direction this way can cooperate with the typical distal settling of the dispensed caused by the action of gravity to create a thorough distribution of the enema composition across the internal surface of the rectum with relatively little solution wasted. For example, enema composition dispensed in a less proximal direction may initially reach a shallower location within the rectum and be drawn out of the patient by gravity, and thereby become wasted.

The lateral dispensing conduits (106) in FIGS. 1A and 1B are rectangular in cross-section when viewed parallel to the longitudinal axis of the elongate member (102).

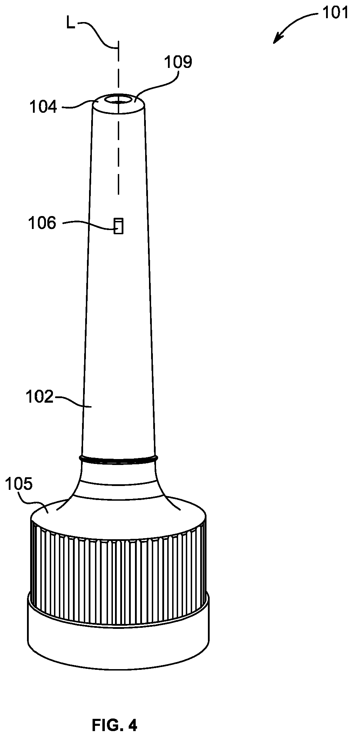

FIG. 4 shows the cannula 101 from a lateral perspective that is normal to the longitudinal axis (L), with one of the lateral dispensing openings (106) facing toward the viewer. The lateral dispensing opening (106) of some embodiments, including the illustrated embodiment is rectangular in shape at the radial exterior of the elongate member (102). The shape of the lateral dispensing opening (106) at the radial exterior of the elongate member (102) is further a non-square rectangle in the illustrated embodiment. The shape of the lateral dispensing opening (106) at the radial exterior of the elongate member (102) is further a rectangle that is narrower in width measured laterally, normal to the longitudinal axis (L), than in height measured longitudinally. In further embodiments, the lateral dispensing openings (106) can have other shapes at the radial exterior of the elongate member (102), such as, for example, a square, a non-square rectangle that is wider laterally than longitudinally, a circle, a diamond, or a triangle. The rectangular shape of the lateral dispensing openings (106) of the illustrated embodiment at the radial exterior of the elongate member (102) is about 1 mm wide, measured normally to the longitudinal axis (L). Further, the rectangular shape of the lateral dispensing openings (106) of the illustrated embodiment at the radial exterior of the elongate member (102) is about 2 mm tall (i.e. in length), measured longitudinally. Thus, the shape of each lateral dispensing opening (106) of the illustrated embodiment at the radial exterior of the elongate member (102) may, in some embodiments, be about twice as tall, measured longitudinally, as it is wide, measured normally to the longitudinal axis (L). Further, the shape of the opening of each lateral dispensing opening (106) may, in some embodiments, have an area of about 2 mm². However, the proportions and area of the lateral dispensing openings (106) at the radial exterior of the longitudinal member (102) may vary in other embodiments. All features illustrated and described herein, and any possible variations thereon, with respect to the shape and size of the lateral dispensing openings (106), may exist in embodiments wherein the lateral dispensing openings (106) have a nozzle shape, such as shown in FIGS. 1A and 1B, or a shape of constant cross-sectional proportions, such as shown in FIGS. 2 and 3A and 3B.

In the embodiments of FIGS. 1A-4, the dispensing conduit (110) can have a cross-sectional area when viewed parallel to the longitudinal axis of the elongate member in the range of from about 0.5 mm² to about 5 mm², such as from about 1 mm² to about 4.5 mm², optionally from about 1.5 mm² to about 3 mm², for example about 2.4 mm². The apical dispensing opening (108) can have a cross-sectional area in the range of from 4 mm² to 12 mm², optionally, from 5 mm² to 10 mm², such as from 5 mm² to 9 mm².., for example about 7 mm².. Thus, a ratio of an area of the outlet of the apical dispensing opening (108) to an area of the outlet of at least one lateral dispensing opening (106) can be from 8:1 to 12:5, and such as from 5:1 to 20:9, and in some embodiments from 10:3 to 9:3, for example about 7:2.4. The elongate member (102) can be from about 3 cm to about 10 cm in length, optionally from about 3.5 cm to about 8 cm in length, and in some embodiments from about 4 cm to about 6 cm in length. At least one lateral dispensing opening (106) can be 0.5 cm to 2.5 cm from the tip (109) of the proximal insertion end (104), and, optionally, at least one lateral dispensing opening (106) is 1 cm to 2 cm from the tip (109) of the proximal insertion end (104). Optionally, two lateral dispensing openings (106) are disposed 1 cm to 2 cm from the tip of the proximal insertion end. The elongate member (102) can be substantially frustoconical in shape. The elongate member (102) can be tapered at an angle of from 0.5 to 15 degrees, such as from 1 to 10 degrees, and in some embodiments from 3 to 8 degrees. Optionally, the elongate member can be tapered from the proximal insertion end to the distal end at an angle of from 0.5 to 15 degrees, such as, from 1 to 10 degrees, and in some embodiments from 3 to 8 degrees. Suitably, when inserted into a patient's rectum in the dispensing position, the at least one lateral dispensing opening is approximately 3 to 6 cm from the anal verge of the patient.

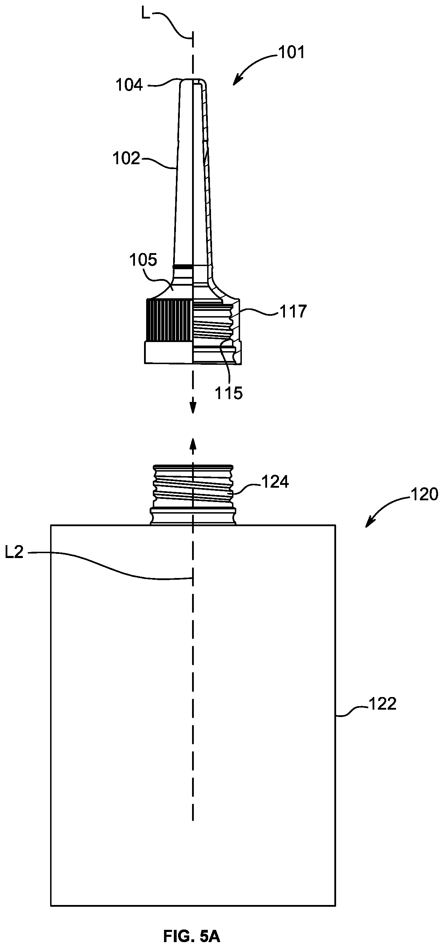

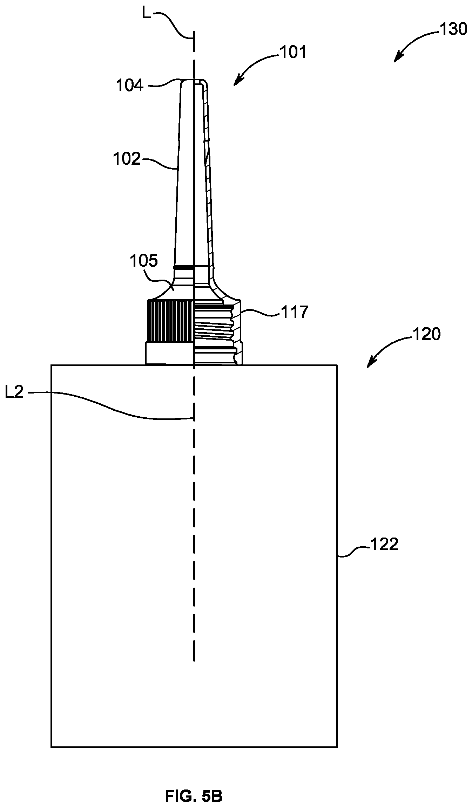

FIG. 5A illustrates the cannula (101) and a reservoir (120) for enema composition prior to assembly. The reservoir (120) includes a body (122) and a finish (124) located at a proximal end of the body (122). The body (122) is hollow and thereby provides a space for storage of enema composition. The body (122) in some embodiments may also be flexible so that it may be compressed by hand. The body can be a compressible portion of a bellow enema bottle. The body (122) in some such embodiments may therefore enable enema composition to be expelled through the finish (124) by manually squeezing the body (122). The cannula (101) may be assembled together with the reservoir (120) to form an assembly (130) as shown in FIG. 5B by aligning the longitudinal axis (L) of the cannula (101) with a longitudinal axis (L2) of the finish (124) and engaging the cuff (117) with the finish (124). In the illustrated embodiment, engaging the cuff (117) with the finish (124) includes rotating the cannula (101) about the longitudinal axis (L) relative to the reservoir (120) to threadedly engage the threading (115) of the cannula (101) with the complementary threading of the finish (124).

The finish (124) may include engagement means complementary to the attachment means of the cannula (101). Thus, in embodiments such as the illustrated embodiment wherein the cannula (101) includes attachment means in the form of threading (115) formed within the cuff (117), the finish (124) may include engagement means in the form of threading complementary to the threading (115) of the cannula (101). However, in other embodiments, the respective attachment and engagement means for securing the cannula (101) to the reservoir (120) may differ. Securing the cannula (101) to the finish (124) may create fluid communication between the interior of the body (122) of the reservoir (120) to the interior of the cannula (101), enabling enema composition to flow from the interior of the body (122) through the lumen (103) of the cannula (101) and out the cannula's dispensing openings (106, 108). In some embodiments, a one way valve, such as a non-return valve or check valve (not shown) may prevent backflow from the cannula to the reservoir. Thus, assembling the cannula (101) with the reservoir (120) as shown in FIG. 5B may establish fluid communication from the interior of the body (122) to the lateral dispensing openings (106) and apical dispensing opening (108) through the finish (124) and lumen (103). In some embodiments, the reservoir (120) may include a non-return valve, such as within the finish (124), to prevent composition from returning from the lumen (103) to the interior of the body (122). The threading or other means provided for securing the cannula (101) to the finish may be fluid tight, or at least substantially fluid tight, such that little or no enema composition may be wasted and so that pressure created within the body (122), such as by squeezing the body (122), may be efficiently transferred to enema composition being dispensed through the dispensing openings (106, 108) of the cannula (101).

Suitably, the reservoir (in form of a bottle or bellows enema bottle) has a volume between 10 mL and 200 mL, between 30 mL and 150 mL, or between 50 mL and 120 mL. In some embodiments, the reservoir has a volume of 30 mL, or 40 mL, or 50 mL, or 60 mL, or 70 mL, or 80 mL, or 90 mL, or 100 mL, or 110 mL, or 120 mL.

In some embodiments, the reservoir (in form of a bottle or bellows enema bottle) can be from 20 to 120 ml, from 70 to 100, or from 80 to 90 ml.

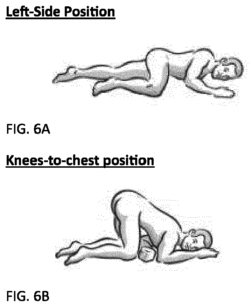

FIG. 6A and 6B show recommended patient positions to adopt for administration of an enema. By adopting such positions, gravity encourages an administered enema composition into the colon. In contrast, administering an enema in a seated position increases the likelihood of leakage.

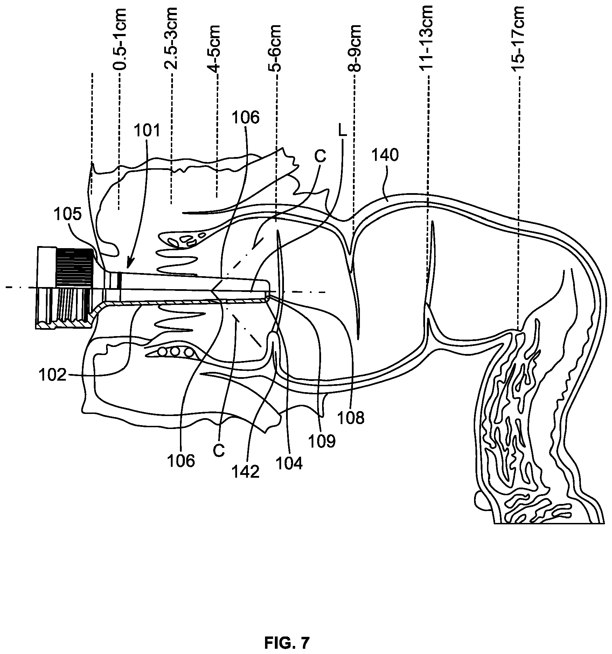

FIG. 7 shows the cannula (101) located at an intended dispensing position within a rectum (140). When the cannula (101) is disposed as shown and assembled together with the reservoir (120) (omitted from FIG. 7 for clarity), the enema composition may be dispensed from the reservoir (120) into the rectum (140) through the cannula (101). When so dispensed, the enema composition shall be directed along the longitudinal axis (L) through the apical dispensing opening (108) and along the central axes (C) through the lateral dispensing openings (106). Thus, the enema composition may be dispensed simultaneously in both a purely proximal component through the apical dispensing opening (108) and at least one partially lateral component through the at least on lateral dispensing openings (106). The dispensing position at which the cannula (101) may be disposed while the enema composition is dispensed may vary depending on the needs of the patient, the size of the cannula (101) used, and the judgment of the clinician/individual administering the enema. However, in at least some embodiments, the cannula may be inserted proximally into the rectum (140) such that the proximal tip (109) of the cannula (101) is at approximately the depth of the inferior rectal valve (142) when the enema composition is dispensed. Further, in at least some embodiments, the lateral dispensing openings (106) may be at a depth of from about 3 cm to 6 cm from the anal verge when the enema composition is dispensed.

Advantageously, the at least one lateral dispensing opening (106), suitably, two lateral dispensing openings (106) may facilitate coating of the interior walls of the rectum to a depth of approximately 8-9 cms from the anus. Suitably, approximately 50 to 70%, such as about 60% (by weight) of an enema composition is administered via the apical dispensing opening and approximately 30 to 50 wt% such as about 40 wt% of the enema composition is administered via the at least one lateral dispensing opening(s). Thus, the lateral dispensing opening(s) (106) facilitate more uniform coating of the enema composition on the interior walls of the lower rectum.

The shape and position of the at least one lateral dispensing opening (106) can give rise to improved delivery in the target sites, such as the rectum and sigmoid/descending colon. The use of conventional cannulas involves spraying a solution from a single apical aperture. When such conventional cannulas are used, the liquid medium flows out of the single aperture and does not cover the wall of the colon uniformly, particularly in the rectum. The presence of the at least one additional lateral apertures in the cannula embodiments described and illustrated herein, as well as their position and size, can improve the delivery of a liquid composition, such as a Rifamycin in situ gelling enema, at the target sites. The cannula of some embodiments described herein combines an apical aperture and at least one additional lateral aperture to allow for delivery in sigmoid/descending colon as well as the rectum.

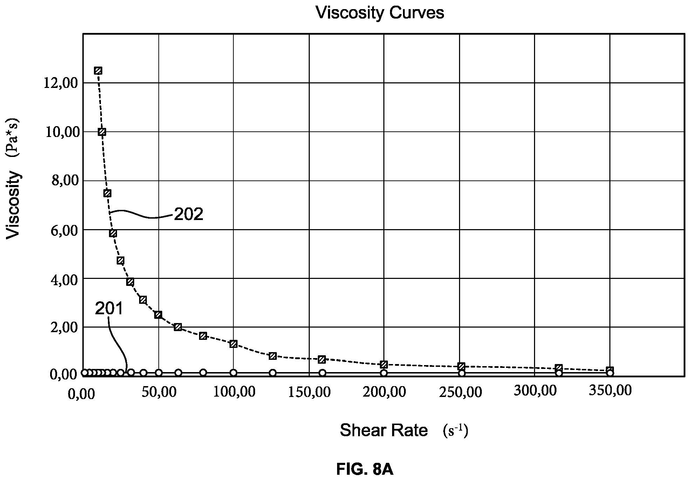

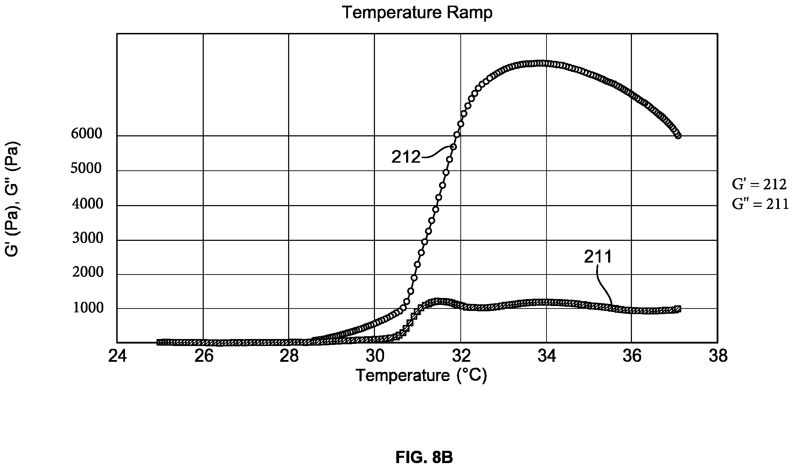

When administered via the cannula of the present disclosure, the enema composition including an active agent, in some embodiments Rifamycin SV, and a polymer mixture as described herein, is automatically heated to body temperature (i.e., about 37 °C). This increase in temperature triggers the transition of the enema composition from a liquid to a gel state (sol-gel transition) or to a structured state due to the thermo-responsive first polymer; then, through the interaction of the ion sensitive second polymer with the ions present in the biorelevant medium and/or environment, said gel or structured state is further strengthened, as demonstrated by an increase in the viscoelastic modulus G'. The change in characteristics of the enema composition from the liquid to the gel state according to some embodiments is illustrated in FIGS. 8A and 8B. As shown in FIG. 8A, a liquid state viscosity curve 201 representing the characteristics of enema composition in the liquid state exhibits relatively little viscosity. The liquid state viscosity curve 201 is consistent with the properties of some enema compositions according to the present disclosure at room temperature, or about 25° C. By contrast, the gel state viscosity curve 202 representing the characteristics of enema composition in the gel state exhibits significantly greater viscosity. The gel state viscosity curve 202 is consistent with the properties of some enema compositions according to the present disclosure at human body temperature, or about 37° C. FIG. 8B illustrates the changes of storage modulus G' (212) and loss modulus G" (211) of an enema composition according to the present disclosure. The shift of modulus G' and G" is from 32°C to 37°C that confirm the change from solution to gel at body temperatures. The transition temperature is about 32° C in the illustrated embodiments, reaching its maximum at 34°C, but may vary in other embodiments. Generally, the transition temperature may be between typical room temperature and typical human body temperature, or the transition temperature may be slightly below typical human body temperature. Thus, the enema composition may tend to remain in the liquid state before application and transition to the gel state after application.

The transition from a liquid form to a gel state or a structured state enhances the bio-adhesiveness of the enema composition, that is driven by the presence of said at least one bioadhesive polymer (third polymer) in the polymer mixture, with a subsequent enhancement of adhesion to the tissues, such as the intestinal or rectal mucosa for a suitable period of time as demonstrated by the muco-adhesion results at 37°C.

In addition, the increase of the viscoelastic modulus of the enema composition slows down the diffusion of the at least one active agent, delaying its delivery to the affected site, with prolongation of the therapeutic effect.

When administered via the cannula of the present disclosure, the application surface area is increased in comparison to conventional cannulas, and the enema compositions described herein are able to form a thin gel or structured layer (state) over the affected area, having a reduced tendency to flow rapidly along the organ walls. The dispensing angle of 30 to 60 degrees ensures a greater application surface area is achieved within the rectum and lower colon, than if a conventional cannula is employed, or for example a cannula having lateral dispensing holes with dispensing angles that are substantially orthogonal to the longitudinal axis of the cannula. The gelation of the enema composition maximizes the contact time between the enema compositions described herein, and the affected area: as a result, the active agent contained therein can remain in contact with the cell membrane of the epithelial cells of the mucosa for a longer period of time, in comparison with other known compositions, and over a greater surface area in comparison to when the same composition is administered via a cannula having only an apical aperture, or a cannula having an apical aperture and lateral apertures with substantially orthogonal dispensing angles therefrom.

The enema composition is able to gel in situ upon contact with the target site of action (distal colon) ensuring the prolongation of the contact time with the mucosa therein ensuring a sustained release of the active agent topically over time. This means that the enema composition is able to provide a composition that becomes, upon application on the intestinal mucosa, a long-lasting gel acting over time at the targeted site. This allows for reduced enema volume.

In some embodiments, such distal part of the gastrointestinal tract includes the distal colon (the descending colon and the sigmoid colon) and the rectum, more specifically the sigmoid colon and rectum. According to such embodiments, such diseases affecting the distal part of the gastrointestinal tract, more specifically the descending colon, the sigmoid colon and the rectum, can be selected from the group including distal ulcerative colitis, proctitis, and proctosigmoiditis. According to an embodiment, such disease affecting the distal part of the gastrointestinal is distal ulcerative colitis, including proctosigmoiditis. According to another embodiment, such disease affecting the distal part of the gastrointestinal tract is proctitis. According to yet another embodiment the disease is pouchitis affecting an ileal pouch.

The foregoing and following examples are included for purpose of illustration of certain aspects and aspects of the disclosure and are not intended to limit the claims.

**TABLE 1: Components for the preparation of example enema composition:**

| **COMPONENTS** | **% w/w** |
|---|---|
| **Rifamycin SV** | **1.00** |
| **Sodium ascorbate** | **0.045** |
| **Ascorbic acid** | **0.040** |
| **Potassium metabisulfite** | **0.012** |
| **Sodium alginate** | **0.200** |
| **Carboxymethyl cellulose sodium** | **0.050** |
| **Poloxamer 407** | **16.50** |
| **Ethanol (96 per cent)** | **1.000** |
| **Purified water** | **Up to 100** |

| | |
|---|---|
| ¹Amount expressed as non-salified rifamycin (i.e., the active moiety). The corresponding amount of rifamycin SV sodium salt is 1.033 % w/w. | |

In a suitable vessel provided with stirrer, purified water (~95% of the total amount) is loaded; then Sodium ascorbate and Ascorbic acid are added under stirring until complete dissolution is achieved. Then Potassium metabisulfite is added to the mixture under stirring until complete dissolution. Then Sodium alginate is added to mixture above under stirring until a homogeneous mixture is achieved. Then, carboxymethyl cellulose sodium is added under stirring until complete dissolution. The solution is cool down below 20°C and then Poloxamer 407 is added the solution is kept under stirring until complete dissolution. Then the pH is checked and correct up to 6.5 with NaOH 1M (or HCl), maintaining the temperature below 20°C. Rifamycin Sodium is added under stirring and maintained under stirring until its complete solubilization. The pH is check and correct it up to 7 with NaOH 1M (or HCl) maintaining the temperature below 20°C. Then Ethanol (96 per cent) is added and stirred until a homogeneous solution is obtained.

Finally, purified water is added up to final weight maintaining stirring.

### Example 2

The composition of Example 2 was prepared as follows as an aqueous solution:

| **COMPONENTS** | **%(W/W)** |
|---|---|
| Rifamycin SV¹ | 0.500 |
| Poloxamer 407 | 16.000 |
| Sodium Alginate | 0.200 |
| Carboxymethylcellulose Sodium | 0.050 |
| Potassium metabisulfite | 0.012 |
| Ascorbic acid | 0.043 |
| Sodium Ascorbate | 0.048 |
| Potassium Sorbate | 0.100 |
| Sodium Benzoate | 0.100 |
| Purified Water | Up to 100 |

| | |
|---|---|
| ¹Amount expressed as non-salified rifamycin (i.e., the active moiety). The corresponding amount of rifamycin SV sodium salt is 0.516 % w/w. | |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then Potassium Metabisulfite is added to the mixture above under stirring until a homogeneous mixture is achieved. Then Potassium Sorbate is added to the mixture above under stirring until a complete dissolution is achieved. Then Sodium Benzoate is added to the mixture above under stirring until a complete dissolution is achieved. Then the pH of the solution is checked and brought it to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then the pH is checked and brought it to pH 7.0, if necessary. The mixture is brought to final weight adding Purified water.

### Example 3

The composition of Example 3 was prepared as follows as an aqueous solution.

| **COMPONENTS** | **%(W/W)** |
|---|---|
| Rifamycin SV¹ | 0.500 |
| Poloxamer 407 | 16.000 |
| Sodium Alginate | 0.200 |
| Carboxymethylcellulose Sodium | 0.050 |
| Potassium metabisulfite | 0.012 |
| Ascorbic acid | 0.043 |
| Sodium Ascorbate | 0.048 |
| Ethanol | 1.000 |
| Purified Water | Up to 100 |

| | |
|---|---|
| ¹Amount expressed as non-salified rifamycin (i.e., the active moiety). The corresponding amount of rifamycin SV sodium salt is 0.516 % w/w. | |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then Potassium Metabisulfite is added to the mixture above under stirring until a homogeneous mixture is achieved. Then the pH of the solution is checked and brought to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then check the pH and bring it to pH 7.0, if necessary. The mixture is brought to final weight adding Purified water.

### Example 4

The composition of Example 4 was prepared as follows:

| **COMPONENTS** | **%(W/W)** |
|---|---|
| Rifamycin SV¹ | 0.500 |
| Poloxamer 407 | 16.000 |
| Sodium Alginate | 0.200 |
| Carboxymethylcellulose Sodium | 0.050 |
| Potassium metabisulfite | 0.012 |
| Ascorbic acid | 0.043 |
| Sodium Ascorbate | 0.048 |
| Ethanol | 2.000 |
| Purified Water | Up to 100 |

| | |
|---|---|
| ¹Amount expressed as non-salified rifamycin (i.e., the active moiety). The corresponding amount of rifamycin SV sodium salt is 0.516 % w/w. | |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then Potassium Metabisulfite is added to the mixture above under stirring until a homogeneous mixture is achieved. Then the pH of the solution is checked and brought to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then the pH is checked and brought to pH 7.0, if necessary. The mixture is brought to final weight adding Purified water.

### Example 5

The composition of Example 5 was prepared as follows:

| **COMPONENTS** | **%(W/W)** |
|---|---|
| Rifamycin SV¹ | 0.500 |
| Poloxamer 407 | 14.00 |
| Gellan Gum | 0.300 |
| Hydroxypropyl cellulose | 0.400 |
| Potassium metabisulfite | 0.012 |
| Ascorbic acid | 0.043 |
| Sodium Ascorbate | 0.048 |
| Purified Water | Up to 100 |

| | |
|---|---|
| ¹Amount expressed as non-salified rifamycin (i.e., the active moiety). The corresponding amount of rifamycin SV sodium salt is 0.516 % w/w. | |

In a suitable vessel provided with a stirrer, Purified water (95% of the total amount) is loaded; then, Hydroxypropyl cellulose is added to the mixture above under stirring until a homogeneous mixture is obtained. Then Ascorbic acid and Sodium Ascorbate are added under stirring until complete dissolution is achieved. Then Potassium Metabisulfite is added to the mixture above under stirring until a homogeneous mixture is achieved. Then the solution is cooled at a temperature ranging between 5 °C and 20 °C; then, poloxamer is added until a homogeneous mixture is achieved. The mixture is kept under stirring until a complete dissolution is achieved and then heated up to room temperature. Then check the pH of the solution and bring it to pH range 6.0-6.5 if needed. Then, Rifamycin SV is added to the mixture above under stirring until complete dissolution is achieved. Then Gellan gum is added to the mixture above under stirring until a homogeneous mixture is achieved. Then the pH is checked and brought to pH 7.0, if necessary. The mixture is brought to final weight adding Purified water.

### Example 6

The composition of Example 6 was prepared as follows as an aqueous solution:

| **COMPONENTS** | **% w/w** |
|---|---|
| Rifamycin SV¹ | 0.500 |
| Poloxamer 407 | 16.000 |
| Sodium Alginate | 0.200 |
| Carboxymethylcellulose Sodium | 0.050 |
| Potassium metabisulfite | 0.012 |
| Ascorbic acid | 0.043 |
| Sodium Ascorbate | 0.048 |
| Purified Water | up to 100.000 |

| | |
|---|---|
| ¹Amount expressed as non-salified rifamycin (i.e., the active moiety). The corresponding amount of rifamycin SV sodium salt is 0.516 % w/w. | |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then Potassium Metabisulfite is added to the mixture above under stirring until a homogeneous mixture is achieved. Then the pH of the solution is checked and brought t to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then check the pH and bring it to pH 7.0, if necessary. The mixture is brought to final weight adding Purified water.

### Example 7

The composition of Example 7 was prepared as follows as an aqueous solution:

| **COMPONENTS** | **% w/w** |
|---|---|
| Rifamycin SV¹ | 0.500 |
| Poloxamer 407 | 16.000 |
| Sodium Alginate | 0.200 |
| Carboxymethylcellulose Sodium | 0.050 |
| Ascorbic acid | 0.040 |
| Sodium Ascorbate | 0.045 |
| Ethanol | 1.000 |
| Purified Water up to | 100.000 |

| | |
|---|---|
| ¹Amount expressed as non-salified rifamycin (i.e., the active moiety). The corresponding amount of rifamycin SV sodium salt is 0.516 % w/w. | |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then the pH of the solution is checked and brought it to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then check the pH and bring it to pH 7.0, if necessary. The mixture is brought to final weight adding Purified water.

### Example 8

The composition of Example 8 was prepared as follows as an aqueous solution:

| **COMPONENTS** | **% w/w** |
|---|---|
| Rifamycin SV¹ | 0.500 |
| Poloxamer 407 | 13.600 |
| Sodium Alginate | 0.100 |
| Carboxymethylcellulose Sodium | 0.050 |
| Ascorbic Acid | 0.040 |
| Sodium Ascorbate | 0.045 |
| Ethanol | 1.000 |
| Purified Water up to | 100.000 |

| | |
|---|---|
| ¹Amount expressed as non-salified rifamycin (i.e., the active moiety). The corresponding amount of rifamycin SV sodium salt is 0.516 % w/w. | |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then the pH of the solution is checked and brought to pH range 6.0-6.5 if needed. Then, Rifamycin is added SV to the mixture above under stirring until complete dissolution is achieved. Then the solution is cooled at a temperature ranging between 5°C and 20°C; then, Poloxamer is added. The mixture is kept under stirring until a complete dissolution is achieved and then heated up to room temperature. Then Ethanol 96% is added to the mixture above under stirring until a homogeneous mixture is achieved. Then the pH is checked and brought to pH 7.0, if necessary. The mixture is brought to final weight adding Purified water.

### Example 9

The composition of Example 9 was prepared as follows as an aqueous solution:

| **COMPONENTS** | **%(W/W)** |
|---|---|
| Rifamycin SV¹ | 0.500 |
| Poloxamer | 14.900 |
| Sodium Alginate | 0.100 |
| Carboxymethylcellulose Sodium | 0.050 |
| Ascorbic acid | 0.040 |
| Sodium Ascorbate | 0.045 |
| Potassium Sorbate | 0.100 |
| Sodium Benzoate | 0.100 |
| Purified Water | Up to 100 |

| | |
|---|---|
| ¹Amount expressed as non-salified rifamycin (i.e., the active moiety). The corresponding amount of rifamycin SV sodium salt is 0.516 % w/w. | |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then, Potassium Sorbate and Sodium Benzoate are added under stirring until complete dissolution is achieved. Then the pH of the solution is checked and brought to pH range 6.0-6.5 if needed. Then, Rifamycin SV is added to the mixture above under stirring until complete dissolution is achieved. Then the solution is cooled at a temperature ranging between 5°C and 20°C; then, Poloxamer is added. The mixture is kept under stirring until a complete dissolution is achieved and then heated up to room temperature. Then Ethanol 96% is added to the mixture above under stirring until a homogeneous mixture is achieved. Then check the pH and bring it to pH 7.0, if necessary. The mixture is brought to final weight adding Purified water.

### Example 10

The composition of Example 10 was prepared as follows as an aqueous solution:

| **COMPONENTS** | **% w/w** |
|---|---|
| Rifamycin SV¹ | 1.000 |
| Poloxamer 407 | 16.000 |
| Sodium Alginate | 0.200 |
| Carboxymethylcellulose Sodium | 0.050 |
| Ascorbic Acid | 0.040 |
| Sodium Ascorbate | 0.045 |
| Ethanol | 1.000 |
| Purified Water | Up to 100.000 |

| | |
|---|---|
| ¹Amount expressed as non-salified rifamycin (i.e., the active moiety). The corresponding amount of rifamycin SV sodium salt is 1.032 % w/w. | |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then the pH of the solution is checked and brought to pH range 6.0-6.5 if needed. Then, Rifamycin is added SV to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then the pH is checked and brought to pH 7.0, if necessary. The mixture is brought to final weight adding Purified water.

### Example 11

The composition of Example 11 was prepared as follows:

| **COMPONENTS** | **% w/w** |
|---|---|
| Rifamycin SV¹ | 0.250 |
| Poloxamer 407 | 15.500 |
| Sodium Alginate | 0.700 |
| Carboxymethylcellulose Sodium | 0.100 |
| Ascorbic Acid | 0.040 |
| Sodium Ascorbate | 0.045 |
| Ethanol | 1.000 |
| Purified Water | Up to 100.000 |

| | |
|---|---|
| ¹Amount expressed as non-salified rifamycin (i.e., the active moiety). The corresponding amount of rifamycin SV sodium salt is 0.258 % w/w. | |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then the pH of the solution is checked and brought to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then the pH is checked and broughtto pH 7.0, if necessary. The mixture is brought to final weight adding Purified water.

### Example 12

The composition of Example 12 was prepared as follows as an aqueous solution:

| **COMPONENTS** | **% w/w** |
|---|---|
| Rifamycin SV¹ | 2.500 |
| Poloxamer 407 | 15.500 |
| Sodium Alginate | 0.300 |
| Carboxymethylcellulose Sodium | 0.100 |
| Ascorbic Acid | 0.040 |
| Sodium Ascorbate | 0.045 |
| Ethanol | 1.000 |
| Purified Water | Up to 100.000 |

| | |
|---|---|
| ¹Amount expressed as non-salified rifamycin (i.e., the active moiety). The corresponding amount of rifamycin SV sodium salt is 2.580 % w/w. | |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then the pH of the solution is checked and brought to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then the pH is checked and brought to pH 7.0, if necessary. The mixture is brought to final weight adding Purified water.

### BIBLIOGRAPHY

1. Fumery, M., et al. (2018). Natural History of Adult Ulcerative Colitis in Population-based Cohorts: A Systematic Review. Clin Gastroenterol Hepatol. 2018 March; 16(3): 343-356.
2. Hoy, SM. (2019) Rifamycin SV MMX: a review in the treatment of traveler's diarrhea. Clin Drug Investig Jul., 39(7):691-697.
3. Rosette, C., et al. (2019). Rifamycin SV exhibits strong anti-inflammatory in vitro activity through pregnane X receptor stimulation and NFkB inhibition. Drug Metabolism and Pharmacokinetics, 34(3): 172-180.
4. Rosette, C. et al. (2013). Anti-inflammatory and immunomodulatory activities of rifamycin SV. Int J Antimicrob Agents 42:182-186.
5. Xie, Y., et al. (2020). Gut epithelial TSC1/mTOR controls RIPK3-dependent necroptosis in intestinal inflammation and cancer. J Clin Invest 130:2111.

### Embodiments

1. A cannula for rectal administration of an enema comprising: an elongate member having a lumen, said elongate member having a proximal insertion end and a distal end, and at least one lateral dispensing opening extending through a sidewall of the elongate member and configured to dispense fluid from the lumen at an angle of from about 30° to about 60° to a longitudinal axis of the elongate member.
2. The cannula of embodiment 1, wherein the at least one lateral dispensing opening is defined by a dispensing conduit in the sidewall of the elongate member, said dispensing conduit extending from an internal opening in an inner surface of the elongate member and an external opening in an outer surface of the elongate member, said dispensing conduit comprising a central axis extending at an angle of from about 30° to about 60° to a longitudinal axis of the elongate member.
3. The cannula of embodiment 2, wherein the central axis extends at an angle of from about 35° to about 55° to the longitudinal axis of the elongate member, preferably from about 40° to about 50° to the longitudinal axis of the elongate member, such as from about 42° to about 48° to the longitudinal axis of the elongate member.
4. The cannula of any preceding embodiment, comprising two or more lateral dispensing openings.
5. The cannula of any preceding embodiment, comprising two or more lateral dispensing openings offset with respect to each other in the longitudinal axis of the elongate member.
6. The cannula of any preceding embodiment, comprising an apical dispensing opening in a tip of the proximal insertion end.
7. The cannula of any preceding embodiment, comprising two lateral dispensing openings, and one apical dispensing opening.
8. The cannula of any preceding embodiment, wherein the external opening of the at least one lateral dispensing opening is circular or non-circular in circumference, preferably, wherein the external opening of at least one lateral dispensing opening is non-circular in circumference.
9. The cannula of any preceding embodiment, wherein the dispensing conduit is polygonal in cross-section when viewed parallel to the longitudinal axis of the elongate member.
10. The cannula of embodiment 9, wherein the dispensing conduit is quadrilateral in cross-section, optionally, rectangular in cross-section when viewed parallel to the longitudinal axis of the elongate member.
11. The cannula of any preceding embodiment, wherein at least one lateral dispensing opening has an internal opening in the inner surface of the elongate member having a circumference greater in length than a circumference of the external opening in the outer surface of the elongate member.
12. The cannula of any preceding embodiment, wherein at least one dispensing opening forms a nozzle through the sidewall extending from the lumen, optionally, wherein the nozzle is a converging nozzle.
13. The cannula of any preceding embodiment, wherein the dispensing conduit has a cross-sectional area when viewed parallel to the longitudinal axis of the elongate member in the range of from about 0.5 mm² to about 5 mm², such as from about 1 mm² to about 4.5 mm², optionally from about 1.5 mm² to about 3 mm², for example about 2.4 mm².
14. The cannula of any preceding embodiment, wherein the elongate member is from about 3 cm to about 10 cm in length, suitably, wherein the elongate member is from about 3.5 cm to about 8 cm in length, optionally, from about 4 cm to about 6 cm in length.
15. The cannula of any preceding embodiment, wherein at least one lateral dispensing opening is 0.5 cm to 2.5 cm from the tip of the proximal insertion end, optionally, wherein at least one lateral dispensing opening is 1 cm to 2 cm from the tip of the proximal insertion end.
16. The cannula of any preceding embodiment, wherein the elongate member is substantially frustoconical in shape.
17. The cannula of any preceding embodiment wherein the proximal insertion end has an external diameter of from 4.5 mm to 7.5 mm in length, optionally of from 5 mm to 7 mm in length, such as from 5.5 mm to 6.5 mm in length.
18. The cannula of any preceding embodiment wherein the distal end of the elongate member has an external diameter of from 7 mm to 14 mm in length, optionally from 8 mm to 12 mm in length, such as from 9 mm to 11 mm in length.
19. The cannula of any preceding embodiment, further comprising a reservoir, optionally, wherein the distal end of the cannula comprises attachment means for attaching the cannula to a reservoir.
20. The cannula of any preceding embodiment, wherein the reservoir is in fluid connection with the lumen of the elongate member, optionally, wherein the cannula or reservoir comprise a one-way valve, such as a check valve or non-return valve.
21. The cannula of embodiment 19 or 20, wherein the reservoir comprises an enema composition.
22. The cannula of embodiment 21, wherein the enema composition is a liquid at room temperature and forms a gel at body temperature.
23. The cannula of embodiment 21 or 22, wherein the enema composition comprises a therapeutic.
24. The cannula of embodiment 23, wherein the enema composition is suitable for treating diseases or conditions of the rectum and/or colon.
25. The cannula of embodiment 24, wherein the diseases of the rectum and/or colon are selected from distal ulcerative colitis (also known as left-sided colitis), proctosigmoiditis, ulcerative proctitis or pouchitis.
26. The cannula of any one of embodiments 21 to 25, wherein the enema composition comprises a therapeutic agent and a polymeric mixture.
27. The cannula of embodiment 26, wherein the the polymeric mixture comprises at least one thermo-responsive polymer (first polymer), at least one ion-sensitive polymer (second polymer) and at least one bio-adhesive polymer (third polymer).
28. The cannula of embodiment 27, wherein the first polymer is selected from: polyoxyethylene-polyoxypropylene block copolymers, such as poloxamer 124, poloxamer 188, poloxamer 237, poloxamer 338, poloxamer 407 and the like, polyethylene glycol)/poly(lactide-coglicolide) block copolymers (PEG-PLGA), polyethylene glycol)-poly(lactic acid)-poly(ethylene glycol) (PEG-PLA-PEG), poly(N-isopropylacrylamide), and cellulose derivatives, like methylcellulose (MC) and hydroxypropylmethylcellulose (HPMC) and the like and mixtures thereof, optionally, the first polymer is a polyoxyethylene-polyoxypropylene block copolymer selected from the group consisting of: poloxamer 124, poloxamer 188, poloxamer 237, poloxamer 338, and poloxamer 407; or a cellulose derivative selected from the group consisting of methylcellulose (MC), hydroxypropylmethylcellulose (HPMC) and mixtures thereof, preferably, the first polymer is polyoxamer 188, poloxamer 407, or mixtures thereof.
29. The cannula of embodiment 27 or 28, wherein the second polymer is a polysaccharide selected from the group comprising but not limited to: carrageenan, gellan gum, pectin, alginate, alginate salts and the like, alginic acid, and mixtures thereof. Preferably, the second polymer is sodium alginate.
30. The cannula of any one of embodiments 27 to 29, wherein the third polymer is selected from: chitosan, hyaluronic acid and salts thereof, cellulose derivatives, such as methyl cellulose, hydroxy propyl methylcellulose, hydroxy propyl cellulose, carboxymethyl cellulose sodium and the like, polyvinyl alcohol, polyvinyl pyrrolidone, polyacrylic acid, polyethylene oxides, cyclodextrins, tragacanth, sodium alginate, xanthan gum, gelatin, pectin, and the like and mixtures thereof, optionally, the third polymer is a cellulose derivative selected from the group consisting of: methyl cellulose, hydroxy propyl methylcellulose, hydroxy propyl cellulose, carboxymethyl cellulose sodium and mixtures thereof, preferably, the third polymer is a sodium salt of carboxymethyl cellulose.
31. The cannula of any one of embodiments 27 to 30, wherein the at least one thermo-responsive polymer is present in an amount of from about 1% to about 25 % by weight with respect to the weight of the enema composition, preferably, the at least one thermo-responsive polymer is present in an amount from about 12% to about 18% by weight with respect to the weight of the enema composition.
32. The cannula of any one of embodiments 27 to 31, wherein the at least one ion-sensitive polymer is present in an amount of from about 0.01% to about 3% by weight with respect to the weight of the enema composition, preferably, the at least one ion-sensitive polymer is present in an amount of from about 0.1% to about 2.0 % by weight with respect to the weight of the enema composition.
33. The cannula of any one of embodiments 27 to 32, wherein the at least one bio-adhesive polymer is present in an amount of from about 0.01% to about 2.0% by weight with respect to the weight of the enema composition, preferably, the at least one bio-adhesive polymer is present in an amount of from about 0.05% to about 0.1% by weight with respect to the weight of the enema composition.
34. The cannula of any one of embodiments 21 to 33, wherein the enema composition further comprises at least one other excipient such as for example antioxidants, chelating agents, preservatives, antimicrobial agents, surfactants, co-surfactants, lipophilic compounds, purified water, organic salts, inorganic salts, buffers agents or mixtures thereof, preferably, the enema composition further comprises at least one antioxidant and a least one preservative.
35. The cannula of any one of embodiments 27 to 34, wherein the therapeutic agent is selected from: rifamycin SV, mesalamine, bethametasone or salts thereof, budesonide, prednisone, prednisolone, cyclosporine A, tofacitinib, optionally, rifamycin SV or a pharmaceutically acceptable salt thereof.
36. The cannula of any one of embodiments 27 to 35, wherein the at least one thermo-responsive polymer (first polymer) comprises poloxamer 407, the at least one ion-sensitive polymer (second polymer) comprises sodium alginate and the at least one bio-adhesive polymer (third polymer) comprises carboxymethyl cellulose.
37. A method of treating ulcerative colitis, particularly distal ulcerative colitis, proctitis and proctosigmoiditis, or pouchitis comprising employing a cannula as defined in any preceding embodiment to rectally administer to a patient in need thereof, an enema composition comprising a therapeutic agent and a polymeric mixture, wherein the therapeutic agent comprises rifamycin SV, or a pharmaceutically acceptable salt thereof, and wherein the enema composition is a liquid at room temperature and forms a gel in situ at body temperature of the patient.
38. A kit of parts comprising:
   (iii) a first package enclosing a cannula as defined in any one of embodiments 1 to 37, and
   (iv) a second gas impermeable package encasing: (a) a reservoir including an enema composition, and (b) an oxygen scavenger, wherein optionally, the second package is an aluminum pouch.
39. A method of administering an enema composition, the method comprising:
   a. disposing the cannula of any one of embodiments 1 to 37 at a dispensing position within a patient's rectum, wherein the cannula is assembled with a reservoir of the enema composition such that the enema composition may travel from the reservoir into the lumen; and
   b. dispensing the enema composition from apical and the lateral dispensing opening while the cannula remains at the dispensing position.
40. The method of embodiment 39, wherein the enema composition is stored as a fluid in the reservoir at a temperature below body temperature prior to the disposing step and the enema composition is configured to transition from the fluid to a gel at body temperature.
41. The method of embodiment 37, 39 or 40, wherein the at least one lateral dispensing opening is located at about 3 cm to about 6 cm from the patient's anal verge when the cannula is at the dispensing position.

## Claims

1. A cannula for rectal administration of an enema comprising: an elongate member having a lumen, said elongate member having a proximal insertion end and a distal end, and at least one lateral dispensing opening extending through a sidewall of the elongate member and configured to dispense fluid from the lumen at an angle of from about 30° to about 60° to a longitudinal axis of the elongate member.

2. The cannula of claim 1, wherein the at least one lateral dispensing opening is defined by a dispensing conduit in the sidewall of the elongate member, said dispensing conduit extending from an internal opening in an inner surface of the elongate member and an external opening in an outer surface of the elongate member, said dispensing conduit comprising a central axis extending at an angle of from about 30° to about 60° to a longitudinal axis of the elongate member.

3. The cannula of claim 2, wherein the central axis extends at an angle of from about 35° to about 55° to the longitudinal axis of the elongate member, preferably from about 40° to about 50° to the longitudinal axis of the elongate member, such as from about 42° to about 48° to the longitudinal axis of the elongate member.

4. The cannula of any preceding claim, comprising an apical dispensing opening in a tip of the proximal insertion end.

5. The cannula of any preceding claim, wherein the dispensing conduit is polygonal in cross-section when viewed parallel to the longitudinal axis of the elongate member, optionally, wherein the dispensing conduit is quadrilateral in cross-section, optionally, rectangular in cross-section when viewed parallel to the longitudinal axis of the elongate member.

6. The cannula of any preceding claim, wherein at least one lateral dispensing opening has an internal opening in the inner surface of the elongate member having a circumference greater in length than a circumference of the external opening in the outer surface of the elongate member.

7. The cannula of any preceding claim, wherein at least one dispensing opening forms a nozzle through the sidewall extending from the lumen, optionally, wherein the nozzle is a converging nozzle.

8. The cannula of any preceding claim, wherein the dispensing conduit has a cross-sectional area when viewed parallel to the longitudinal axis of the elongate member in the range of from about 0.5 mm² to about 5 mm², such as from about 1 mm² to about 4.5 mm², optionally from about 1.5 mm² to about 3 mm², for example about 2.4 mm².

9. The cannula of any preceding claim, wherein the elongate member is from about 3 cm to about 10 cm in length, suitably, wherein the elongate member is from about 3.5 cm to about 8 cm in length, optionally, from about 4 cm to about 6 cm in length.

10. The cannula of any preceding claim, wherein at least one lateral dispensing opening is 0.5 cm to 2.5 cm from the tip of the proximal insertion end, optionally, wherein at least one lateral dispensing opening is 1 cm to 2 cm from the tip of the proximal insertion end.

11. The cannula of any preceding claim, further comprising a reservoir, optionally, wherein the distal end of the cannula comprises attachment means for attaching the cannula to a reservoir.

12. The cannula of claim 11, wherein the reservoir comprises an enema composition, optionally, wherein the enema composition is suitable for treating diseases or conditions of the rectum and/or colon, further optionally, wherein the diseases of the rectum and/or colon are selected from distal ulcerative colitis (also known as left-sided colitis), proctosigmoiditis, ulcerative proctitis or pouchitis.

13. The cannula of claim 12, wherein the enema composition is a liquid at room temperature and forms a gel at body temperature.

14. The cannula of any one of claims 12 or 13, wherein the enema composition comprises a therapeutic agent and a polymeric mixture.

15. The cannula of claim 14, wherein the polymeric mixture comprises at least one thermo-responsive polymer (first polymer), at least one ion-sensitive polymer (second polymer) and at least one bio-adhesive polymer (third polymer).

16. The cannula of claim 15, wherein the first polymer is selected from: polyoxyethylene-polyoxypropylene block copolymers, such as poloxamer 124, poloxamer 188, poloxamer 237, poloxamer 338, poloxamer 407 and the like, polyethylene glycol)/poly(lactide-coglicolide) block copolymers (PEG-PLGA), polyethylene glycol)-poly(lactic acid)-poly(ethylene glycol) (PEG-PLA-PEG), poly(N-isopropylacrylamide), and cellulose derivatives, like methylcellulose (MC) and hydroxypropylmethylcellulose (HPMC) and the like and mixtures thereof, optionally, the first polymer is a polyoxyethylene-polyoxypropylene block copolymer selected from the group consisting of:
poloxamer 124, poloxamer 188, poloxamer 237, poloxamer 338, and poloxamer 407; or a cellulose derivative selected from the group consisting of methylcellulose (MC), hydroxypropylmethylcellulose (HPMC) and mixtures thereof, preferably, the first polymer is polyoxamer 188, poloxamer 407, or mixtures thereof; and/or
wherein the second polymer is a polysaccharide selected from the group comprising but not limited to: carrageenan, gellan gum, pectin, alginate, alginate salts and the like, alginic acid, and mixtures thereof, preferably, the second polymer is sodium alginate; and/or
wherein the third polymer is selected from: chitosan, hyaluronic acid and salts thereof, cellulose derivatives, such as methyl cellulose, hydroxy propyl methylcellulose, hydroxy propyl cellulose, carboxymethyl cellulose sodium and the like, polyvinyl alcohol, polyvinyl pyrrolidone, polyacrylic acid, polyethylene oxides, cyclodextrins, tragacanth, sodium alginate, xanthan gum, gelatin, pectin, and the like and mixtures thereof, optionally, the third polymer is a cellulose derivative selected from the group consisting of: methyl cellulose, hydroxy propyl methylcellulose, hydroxy propyl cellulose, carboxymethyl cellulose sodium and mixtures thereof, preferably, the third polymer is a sodium salt of carboxymethyl cellulose.

17. The cannula of any one of claims 15 or 16, wherein the at least one thermo-responsive polymer is present in an amount of from about 1% to about 25 % by weight with respect to the weight of the enema composition, preferably, the at least one thermo-responsive polymer is present in an amount from about 12% to about 18% by weight with respect to the weight of the enema composition;
and/or wherein the at least one ion-sensitive polymer is present in an amount of from about 0.01% to about 3% by weight with respect to the weight of the enema composition, preferably, the at least one ion-sensitive polymer is present in an amount of from about 0.1% to about 2.0 % by weight with respect to the weight of the enema composition; and/or
wherein the at least one bio-adhesive polymer is present in an amount of from about 0.01% to about 2.0% by weight with respect to the weight of the enema composition, preferably, the at least one bio-adhesive polymer is present in an amount of from about 0.05% to about 0.1% by weight with respect to the weight of the enema composition.

18. The cannula of any one of claims 15 to 17, wherein the at least one thermo-responsive polymer (first polymer) comprises poloxamer 407, the at least one ion-sensitive polymer (second polymer) comprises sodium alginate and the at least one bio-adhesive polymer (third polymer) comprises carboxymethyl cellulose.

19. The cannula of any one of claims 14 to 18, wherein the therapeutic agent is selected from: rifamycin SV, mesalamine, bethametasone or salts thereof, budesonide, prednisone, prednisolone, cyclosporine A, tofacitinib, optionally, wherein the therapeutic agent is rifamycin SV or a pharmaceutically acceptable salt thereof.

20. A method of treating ulcerative colitis, particularly distal ulcerative colitis, proctitis and proctosigmoiditis, or pouchitis comprising employing a cannula as defined in any preceding claim to rectally administer to a patient in need thereof, an enema composition comprising a therapeutic agent and a polymeric mixture, wherein the therapeutic agent comprises rifamycin SV, or a pharmaceutically acceptable salt thereof, and wherein the enema composition is a liquid at room temperature and forms a gel in situ at body temperature of the patient.

21. A kit of parts comprising:
(i) a first package enclosing a cannula as defined in any one of claims 1 to 19, and
(ii) a second gas impermeable package encasing: (a) a reservoir including an enema composition, and (b) an oxygen scavenger, wherein optionally, the second package is an aluminum pouch.

22. A method of administering an enema composition, the method comprising:
a. disposing the cannula of any one of claims 1 to 19 at a dispensing position within a patient's rectum, wherein the cannula is assembled with a reservoir of the enema composition such that the enema composition may travel from the reservoir into the lumen; and
b. dispensing the enema composition from apical and the lateral dispensing openings while the cannula remains at the dispensing position.

23. The method of claim 22, wherein the enema composition is stored as a fluid in the reservoir at a temperature below body temperature prior to the disposing step and the enema composition is configured to transition from the fluid to a gel at body temperature.

24. The method of claim 20, 22 or 23, wherein the at least one lateral dispensing opening is located at about 3 cm to about 6 cm from the patient's anal verge when the cannula is at the dispensing position.
